# EUROPEAN PATENT SPECIFICATION

(11) **EP 1 326 974 B1**
(45) Date of publication and mention of the grant of the patent: **27.12.2006**
(21) Application number: 01977082.5
(22) Date of filing: 28.09.2001
(51) Int. Cl.: C12N 15/12, C07K 14/54, C07K 16/24, A61K 38/20, G01N 33/50

(54) **METHODS OF USING A HUMAN IL-17-RELATED POLYPEPTIDE TO TREAT DISEASE**
VERFAHREN ZUR VERWENDUNG EINES HUMANEN IL-17 VERWANDTEN POLYPEPTIDS ZUR BEHANDLUNG VON KRANKHEITEN
PROCEDES RELATIFS A L'UTILISATION DE POLYPEPTIDE LIE A L'INTERLEUKINE 17 (IL-17) HUMAINE POUR LE TRAITEMENT DE MALADIES-

(30) Priority: 13.10.2000 US 240177 P; 03.08.2001 US 309936 P
(43) Date of publication of application: 16.07.2003
(73) Proprietor: ELI LILLY AND COMPANY, Indianapolis, IN 46285 (US)
(72) Inventor: GLASEBROOK, Andrew, Lawrence, Zionsville, IN 46077 (US); LIU, Ling, Carmel, IN 46032 (US); NEWTON, Christy, Michelle, Indianapolis, IN 46259 (US); TETREAULT, Jonathan Wendell, Indianapolis, IN 46202 (US)
(74) Representative: Kent, Lindsey Ruth
(86) International application number: PCT/US2001/027737
(87) International publication number: WO 2002/033083

(56) References cited:
- WO-A-00/20593
- WO-A-01/46420
- WO-A-01/59120
- WO-A-02/08285
- WO-A-02/10393
- WO-A-99/60127
- WO-A-99/61617

## Description

### FIELD OF THE INVENTION

The invention relates to the treatment or prevention of mammalian disorders associated with endothelial cell apoptosis or mediated through inflammation. More particularly, the invention relates to the treatment or prevention of sepsis, septic shock, systemic inflammatory response syndrome, acute respiratory distress syndrome (ARDS), atherosclerosis, hypertension, congestive heart failure, primary pulmonary hypertension and allograft vasculopathy.

### BACKGROUND OF THE INVENTION

Endothelial cells lining the vascular lumen have a central role in the modulation of vascular tone, coagulation, permeability, blood flow, angiogenesis, and thrombolysis. The maintenance of homeostasis of the vascular endothelial cell population includes a balance of proliferation, necrosis, and apoptosis.

The process of apoptosis, or programmed cell death, is a genetically controlled process of cell death by which different stimuli activate a cascade of cellular proteases. These proteases, known as caspases, specifically cleave cellular structures towards disassembling a cell into small apoptotic bodies without release of cellular contents or damage to surrounding cells. Apoptosis of endothelial cells leads to an alteration of endothelial function through the distortion of endothelial monolayer architecture as a consequence of the altered shape and reduced size of apoptotic cells. Apoptosis of endothelial cells may be induced by any of a number of environmental stimuli, including radiation, high glucose, removal of extracellular matrix, and detachment of cells from the basal membrane (Fujita et al., Int. Arch. of Allergy and Immunol. 117:202 (1998)).

Apoptosis in endothelial cells potentially may be stimulated either directly or indirectly by lipopolysaccharide ("LPS"). LPS, a known endotoxin, is a component of the outer membrane of gram negative bacteria. In addition, pathogenic bacteria, viruses, and plants liberate lipopolysaccharide-inducing substances. LPS is the major mediator in the development of endotoxin-induced shock. The chemical structures of LPS molecules obtained from different bacteria may vary in a species-specific fashion; however, the region called the lipid A region is common to all LPS molecules (Rietschel, E., *et al.,* in Handbook of Endotoxins, Elsevier, 1:187-214 (1984)). The lipid A region mediates many, if not all, of the LPS-dependent pathophysiologic changes that characterize sepsis and gram negative bacteremia. LPS is a primary cause of death in humans afflicted with gram-negative sepsis (van Deventer et al., Lancet, 1:605 (1988); Ziegler *et al.,* J. Infect. Dis. 136:19-28 (1987)). LPS released from gram-negative bacteria infection may also play a role in the pathology of autoimmune conditions such as Reiter's syndrome, which is associated with rheumatoid arthritis.

LPS challenge causes endothelial cells, polymorphonuclear leukocytes, and cells of the monocyte/macrophage lineage to rapidly release a variety of cell products, including immunoregulatory substances that are capable of initiating, modulating, or mediating humoral and cellular immune responses and processes. Studies have shown that LPS induces monocytes/macrophages to release inflammatory cytokines such as TNF-α, IL-1, IL-6, and IL-12 which play a major role in the cascade of events leading to endotoxic shock.

Tumor necrosis factor (TNF) is apparently a primary mediator of septic shock (Beutler *et al.*, N. Eng. J. Med. 316:379 (1987)). Intravenous injection of LPS into animals and man produces a rapid, transient release of TNFα (Beutler *et al.*, Science, 229:869 (1985); Mathison et *al.,* J. Clin. Invest. 81:1925 (1988)). One of the many cellular responses activated upon the release of TNFα and its subsequent binding to TNFα cellular receptors is apoptosis. Several studies have shown that TNFα directly induces endothelial cell apoptosis (Fujita et al., Int. Arch. of Allergy and Immunol. 117:202 (1998)). *In vitro,* many human endothelial cells are susceptible to apoptosis by TNFα only in the presence of RNA transcription inhibitors or protein synthesis inhibitors (such as cycloheximide). Human umbilical vein epithelial cell (HWEC) survival following exposure to TNFα, IL-1, or LPS, or is dependent on synthesis of cytoprotective proteins. Protein synthesis inhibitors prevent synthesis of these proteins after exposure to TNFα, IL-1, or LPS (Zen et al., J. Biol. Chem. 274:28808 (1999); Polunovsky et al., Exp. Cell Res. 214:584 (1994)). It has also been shown that a TNF-binding protein can protect mice against LPS-induced death as well as endothelial cell apoptosis (Hamimovitz-Friedman et al., J. Exp. Med. 186:1831 (1997)). The induction of TNFα in response to LPS is therefore thought to play a central role in the apoptosis of endothelial cells.

In addition to TNF-α, interferon-γ (IFN-γ) and IL-12 also contribute to LPS induced sepsis (Ozmen, L., et al., J. Exp. Med. 180:907, (1994)). IFN-γ is secreted by T cells and NK cells. The immunomodulatory effects of IFN-γ are extensive and diverse. In monocyte/macrophages, the activities of IFN-γ include: increasing the expression of class I and II MHC antigens; increasing the production of IL-1, platelet-activating factor and H₂O₂; protection of monocytes against LAK cell-mediated lysis; downregulation of IL-8 mRNA expression that is upregulated by IL-2; and, with LPS, induction of NO production (Billiau, A. and R. Dijkmans, Biochem. Pharmacol. 40:1433 (1990); Sen, G.C. and P. Lengyel, J. Biol. Chem. 267:5017 (1992); Gusella, G.L. et al., J. Immunol. 151:2725 (1993); Bulut, V. et al., Biochem. Biophys. Res. Commun. 195:1134 (1993)). IFN-γ has also been demonstrated to be chemotactic for monocytes but not neutrophils (Issekutz, A.C. and T.B. Issekutz, J. Immunol. 151:2105 (1993)). IFN-γ selectively enhances both IgG2a secretion by LPS-stimulated B cells and IgG3 secretion in T cell independent type 2 antigen-mediated B cell activation (Snapper, C.M. *et al.,* J. Exp. Med. 175:1367 (1992); Snapper, C.M. *et al.,* J. Immunol. 140:2121 (1988)). It has also been reported to induce its own expression (Halloran, P.F. *et al*., J. Immunol. 148:3837 (1992)). IFN-γ has been shown to upregulate ICAM-1, but not E-Selectin or VCAM-1, expression on endothelial cells (Thornhill, M.H. *et al.,* Scand. J. Immunol. 38:279 (1993)). Moreover, IFN-γ has been shown to contribute to the Swan reaction induced by gram-negative bacteria (Ogasawara, K. et. al., J. Immuno. 160:3522 (1998)). IFN-γ stimulates macrophages and monocytes to secret TNF-α and in turn up-regulates TNF-α receptor expression.

In contrast to IFN-γ, IL-12 is produced by macrophages and B-lymphocytes. IL-12 has been shown to have multiple effects on T cells and NK cells (D'Andrea, A. *et al.,* J. Exp. Med. 176:1387 (1992); Chan, S.H. *et al.,* J. Exp. Med. 173:869 (1991)). These effects include stimulation of production of IFN-γ and TNF by resting and activated T and NK cells, synergizing with other IFN-γ inducers at both the transcriptional and post-transcriptional levels to induce IFN-γ gene expression, enhancing the cytotoxic activity of resting NK and T cells, inducing and synergizing with IL-2 in the generation of lymphokine-activated killer (LAK) cells, acting as a co-mitogen to stimulate proliferation of resting T cells, and inducing proliferation of activated T and NK cells (D'Andrea, *et al.,* (1992)). Evidence indicates that IL-12, produced by macrophages in response to infectious agents, is a central mediator of the cell-mediated immune response by its actions on the development, proliferation, and activities of Th1 cells (Locksley, R.M., Proc. Natl. Acad. Sci. (USA), 90:5879 (1993); Trinchieri, G., Immunol. Today 14:335 (1993); Scott, P., Science, 260:496 (1993); Hseih, C.S. et *al.,* Science 260:547 (1993)). These IL-12 activities are antagonized by factors which are associated with the development of uncommitted T helper cells into Th2 cells and mediation of the humoral immune response (e.g., IL-4 and IL-10; Locksley, R.M., Proc. Natl. Acad. Sci. (USA) 90:5879 (1993); Trinchieri, G., (1993); Scott, P., Science 260:496, (1993); and Hseih, C.S. *et al*., Science 260:547(1993)).

In addition to sepsis, both IFN-γ and IL-12 are upregulated in many other inflammatory diseases. For instance, it has been shown that antibodies against IL-12 can prevent superantigen-induced and spontaneous relapses of experimental autoimmune encephalomyelitis (EAE), a T cell-mediated autoimmune disease of the central nervous system that serves as a model for multiple sclerosis (Constantinescu, C.S. *et al.,* J. Immunology, 161:5097 (1998)). It also has been reported that blocking IFN-γ production in T cells through the use of anti-IL-18 antibodies can impede the development of experimental autoimmune encephalomyelitis (Zamvil, S.S. et al., Annu. Rev. Immunol. 8:579 (1990)).

Type I diabetes is also considered an autoimmune disease. More specifically, it is characterized by a spontaneous mononuclear infiltration of the pancreas. The progression of the disease toward intra-insulitis correlates with an increase in Th1 cells and subsequent loss of β cells which results in insulin deficiency (Shehedeh, N.N. et al., J. Autoimmun., 6:291-300 (1993); Rothe, H., *et al.*, Diabetologia, 37:1154-1158 (1994)). The destructive effects of IFN-γ produced by these T cells has been alleviated by using neutralizing antibodies to IFN-γ or IL-12 (Debray-Sachs, M. *et al*., J. Autoimmun., 4:237-248 (1994)).

Synergism between IL-12 and IL-18 is important to the production of IFN-γ from T cells and NK cells, which sustain inflammation (Micallef, M. et al., J. Immunol., 26:1647-1651 (1996)). In addition to stimulation of IFN-γ secretion, IL-12 also increases expression of the IL-18 receptor on Th0 cells and B-cells. It has been shown that IL-18 is produced by articular chondrocytes and induces proinflammatory and catabolic responses, and increased production of IL-18 was found in synovium of patients with rheumatoid arthritis (Tsaiwei, O., J. Immunol. 162:1096-1100 (1999); Arthritis Rheum. 40:274 (1997)).

IL-17 is another known proinflammatory molecule. It is produced by activated T lymphocytes, primarily by memory T cells (Rouvier, E. *et al*., J. Immunol., 150:5445 (1993); Yao, Z. *et al.,* J. Immunol., 155:5483 (1995); Kennedy, J., *et al.*, J. Interferon Cytokine Res., 16:611 (1996); Fossiez, F. *et al.,* J. Exp. Med., 183:2593 (1996)). IL-17 appears to mediate communication between the immune system and the hematopoietic system. T cell derived IL-17 induces fibroblasts to secrete IL-6, IL-8, ICAM-1, and G-CSF, apparently by an NF-kB-mediated mechanism (Yao, Z. *et al.*, Immunity, 3:811 (1995)). IL-6 in turn promotes development of granulocyte/macrophage colonies, and G-CSF directs development of neutrophils (Fossiez, F. *et al.*, J. Exp. Med. 183:2593 (1996); Ikebuchi, K. *et al*., Proc. Natl. Acad. Sci. (USA), 84:9035 (1987); Berliner, N., *et al*., Bloo, 85:799 (1995); Roberts, A.W. and Metcalf, D., Exp. Hematol. 22:1156 (1994); Broxmeyer, H.E., J. Exp. Med., 183:2411 (1996)). IL-17 also enhances proliferation of partially activated T cells, and it upregulates nitric oxide production in osteoarthritic cartilage (Yao, Z. *et al.,* Immunity 3:811 (1995); Attur, M.G. *et al.*, Arthritis Rheum. 40:1050 (1997)).

In contrast to the cytokines mentioned thus far, IL-10 is an anti-inflammatory cytokine. IL-10 is a pleiotrophic cytokine that inhibits the production of a number of cytokines (including IL-1, GM-CSF, TNF, IL-6, IL-8, IL-10, IL-12, and IFN-γ) by activated Th-1 cells, NK cells, and monocyte/macrophages. IL-10 has also been shown to inhibit macrophage cytotoxic activity and to stimulate the proliferation and differentiation of B cells, mast cells, and thymic T cells (Moore, K.W. *et al.*, Annu. Rev. Immunol., 11:165 (1993); Fiorentino, D.F. *et al.,* J. Exp. Med., 170:2081 (1989); Mosmann, T.R., Adv. Immunol., 56:1 (1994)).

A new member of the interleukin superfamily, IL-17C, was reported in WO 99/60127 and by Li, et al. (Li, et al., PNAS, 97(2):773-778). An identical sequence was disclosed as IL-21 in WO 99/61617. Despite the *in silico* identification of the polypeptide sequence of this cytokine the therapeutic utility of exogenous administration of either the poplypeptide itself, or agonists or antagonists thereto, remained unresolved in the art.

### BRIEF SUMMARY OF THE INVENTION

The present invention relates generally to methods and therapies for effectively preventing or treating atherosclerosis, hypertension, congestive heart failure, primary pulmonary hypertension, sepsis, gram negative bacteremia, allergic autoimmune diseases, type 1 diabetes, inflammation, rheumatoid arthritis, ARDS, allograft vasculopathy, and conditions or symptoms related thereto, by administering functional LP-48 polypeptides and thereby intervening in the underlying mechanisms of these disorders.

In one aspect, the present invention provides the use of a polypeptide comprising amino acids 19-197 of the LP-48 as shown in SEQ ID NO: 2 in the manufacture of a medicament for treating or preventing an inflammation mediated disorder in a mammal, wherein said disorder is selected from sepsis, septic shock, systemic inflammatory response syndrome and acute respiratory distress syndrome (ARDS).

In another aspect, the present invention provides the use of a polypeptide comprising amino acids 19-197 of the LP-48 as shown in SEQ ID NO: 2 in the manufacture of a medicament for treating or preventing a disorder associated with endothelial cell apoptosis in a mammal, wherein said disorder is selected from atherosclerosis, hypertension, congestive heart failure, primary pulmonary hypertension, acute respiratory distress syndrome (ARDS) and allograft vasculopathy.

Preferred polynucleotides for practicing the present invention are those that encode the full-length LP-48 polypeptide as shown in SEQ ID NO:2. More preferred polynucleotides are those that encode the LP-48 mature polypeptide as represented by amino acids 19-197 of SEQ ID NO:2. Most preferred polynucleotides for practicing the present invention are those which comprise LP-48 encoding polynucleotides joined in-frame to additional polynucleotide sequences which encode non LP-48 amino acid sequences.

A preferred polypeptide for practicing the present invention is the full-length LP-48 polypeptide as shown in SEQ ID NO:2. A more preferred polypeptide is the LP-48 mature polypeptide as represented by amino acids 19-197 of SEQ ID NO:2. The most preferred polypeptides for practicing the present invention are LP-48 fusion proteins which comprise a LP-48 polypeptide or a fragment thereof joined to additional amino acids sequences of non-LP-48 polypeptides. In particular, the LP-48 fusion protein for use in the invention may comprise amino acids 19-197 of the LP-48 as shown in SEQ ID NO:2 or an amino acid sequence as shown in SEQ ID NO:2, and an immunoglobulin constant domain sequence.

### DETAILED DESCRIPTION OF THE INVENTION

### Definitions

The term "LP-48" refers to either the nucleic acid sequence, SEQ ID NO:1, or the amino acid sequence, SEQ ID NO:2, of a member of the interleukin superfamily. LP-48 has been reported as IL-17C (WO 99/60127; Li, et al., PNAS, 97(2):773-778), and also appears in the GenBank database as accession no. AF152099. An identical sequence also appears as IL-21 in WO 99/61617.

The LP-48 gene (SEQ ID NO:1) comprises a single large open reading frame encoding a polypeptide of 197 amino acids (SEQ ID NO:2). It is expected that expression of SEQ ID NO:1 *in vivo* or by mammalian cells *in vitro* would result in a mature 179 amino acid protein. Such mature protein is included within the definition of "LP-48" or "LP-48 polypeptide".

LP-48 sequences can be isolated from nature or can be produced by recombinant or synthetic means. The phrase "LP-48 polypeptide", as used herein, is meant to include, but is not limited to, glycosylated, unglycosylated, and/or modified forms of the LP-48 polypeptide as shown in SEQ ID NO:2 (or the mature LP-48 polypeptide) further comprising one or more conservative substitutions of, additions to, or deletions of, the amino acid sequence as shown in SEQ ID NO:2 provided that said modified forms of the LP-48 polypeptide demonstrate substantially similar biological activity as the LP-48 polypeptides described herein in at least one of the assays exemplified herein. The term "activity" or the phrase "biological activity" in reference to a LP-48 polypeptide concerns the capacity of a LP-48 polypeptide to induce, *in vivo* and/or *in vitro,* one or more of the biological consequences attributed to LP-48 polypeptides by the present disclosure. Accordingly, activity of LP-48 polypeptides can be assessed by one or more of the *in vitro* or *in vivo* assays exemplified herein. A preferred biological activity includes, for example, the ability to protect against LPS induced septic shock as determined by the methodology described in Example 12.

The term "LP-48 polypeptide" is also meant to encompass polypeptides containing pro-, or prepro-sequences, that when processed result in the production of a LP-48 polypeptide or fragment thereof. The natural leader sequence of human LP-48, as represented by amino acids 1-18 of SEQ ID NO:2, is an example of such a pro-sequence that would when processed result in the production of a LP-48 polypeptide or fragment thereof.

Similarly, LP-48 polynucleotides or polypeptides useful to practice the present invention may additionally contain other non-LP-48 polynucleotide or polypeptide sequences, respectively, provided that the polypeptide encoded thereby still retains an essential functional LP-48 activity as disclosed herein. More specifically, LP-48 polypeptides useful in practicing the present invention also includes chimeric protein molecules not found in nature comprising a translational fusion, or in some cases an enzymatic fusion, in which two or more different proteins or fragments thereof are covalently linked on a single polypeptide chain. A preferred LP-48 polypeptide for practicing the present invention comprises at least one functional fragment of the full-length LP-48 polypeptide as shown in SEQ ID NO:2 and at least one effector function of an immunoglobulin constant domain. The fusion molecules are a subclass of chimeric polypeptide fusions of LP-48 polypeptides that additionally contain a portion of an immunoglobulin sequence (herein referred to LP-48-Ig). The chimeric LP-48-Ig fusions may also comprise forms in monomeric, homo- or heteromultimeric, and particularly homo- or heterodimeric, or homo- or heterotetrameric forms; optionally, the chimeras may be in dimeric forms or homodimeric heavy chain forms. Tetrameric forms containing a four chain structural unit are the natural forms in which IgG, IgD, and IgE occur. A four-chain structure may also be repeated. Different chimeric forms containing a native immunoglobulin are known in the art (WO 98/25967). The mature human protein of Example 12 is exemplary of a "LP-48-Ig." As used herein, the term "LP-48-Ig" designates antibody-like molecules that combine at least one LP-48 domain with the effector functions of immunoglobulin constant domain. The immunoglobulin constant domain sequence may be obtained from any immunoglobulin, such as IgG-1, IgG-2, IgG-3 or IgG-4 subtypes, IgA (including IgG-1 and IgA-2), IgE, IgD or IgM. Preferred fusions contain the LP-48 fused to the amino terminus of the Ig region. However, fusions of an LP-48 polypeptide or fragment thereof to the C-terminus of the Ig region are also contemplated.

LP-48 fusion polypeptides can also comprise additional amino acid residues, such as affinity tags that aid in the purification or identification of the molecule or provide sites of attachment to a natural ligand. These additional amino acid residues are typically placed at, but are not limited to, the N- or C-terminus of the LP-48 polypeptide.

The term "epitope tagged" where used herein refers to a chimeric polypeptide comprising an LP-48 polypeptide, or domain sequence thereof, fused to a "tag polypeptide". The tag polypeptide has enough residues to provide an epitope against which an antibody may be made, or which can be identified by some other agent, yet is short enough such that it does not interfere with the activity of the LP-48 polypeptide. The tag polypeptide preferably is also fairly unique so that the antibody does not substantially cross-react with other epitopes. Suitable tag polypeptides generally have at least six amino acid residues and usually between about 8 to about 50 amino acid residues (preferably, between about 10 to about 20 residues).

The term "amino acid" is used herein in its broadest sense, and includes naturally occurring amino acids as well as non-naturally occurring amino acids, including amino acid analogs and derivatives. The latter includes molecules containing an amino acid moiety. One skilled in the art will recognize, in view of this broad definition, that reference herein to an amino acid includes, for example, naturally occurring proteogenic L-amino acids; D-amino acids; chemically modified amino acids such as amino acid analogs and derivatives; naturally occurring non-proteogenic amino acids such as norleucine, β-alanine, ornithine, etc.; and chemically synthesized compounds having properties known in the art to be characteristic of amino acids.

The incorporation of non-natural amino acids, including synthetic non-native amino acids, substituted amino acids, or one or more D-amino acids into a LP-48 analog ("D- LP48 polypeptides") is advantageous in a number of different ways. D-amino acid-containing polypeptides exhibit increased stability *in vitro* or *in vivo* compared to L-amino acid-containing counterparts. Thus, the construction of polypeptides incorporating D-amino acids can be particularly useful when greater stability is desired or required in vivo. More specifically, D-peptides are resistant to endogenous peptidases and proteases, thereby providing improved bioavailability of the molecule, and prolonged lifetimes *in vivo* when such properties are desirable. When it is desirable to allow the peptide to remain active for only a short period of time, the use of L-amino acids therein will permit endogenous peptidases, proteases to digest the molecule, thereby limiting the cell's exposure to the molecule. Additionally, D-peptides cannot be processed efficienty for major histocompatibility complex class II-restricted presentation to T helper cells, and are therefore less likely to induce humoral immune responses in the whole organism.

In addition to using D-amino acids, those of ordinary skill in the art are aware that modifications in the amino acid sequence of a peptide, polypeptide, or protein can result in equivalent, or possibly improved, second generation peptides that display equivalent or superior functional characteristics when compared to the original amino acid sequences. Alterations in the LP-48 polypeptides of the present invention resulting in LP-48 analogs can include one or more amino acid insertions, deletions, substitutions, truncations, fusions, shuffling of subunit sequences, and the like, either from human manipulation, provided that the sequences produced by such modifications have substantially the same (or improved or reduced, as may be desirable) activity(ies) as the LP-48 polypeptide sequences disclosed herein. The term "LP-48 analog" refers to any modified form of a LP-48 polypeptide that exhibits substantially the same or enhanced biological activity in vivo and/or *in vitro* as compared to the corresponding unmodified form and is pharmaceutically more desirable, in at least one aspect, as compared to the corresponding unmodified LP-48 polypeptide.
As used herein, the term "LP-48 analog" is intended to encompass LP-48 polypeptides as defined herein wherein the LP-48 polypeptide further comprises at least one modification not normally native to LP-48 polypeptides. The term "modification" includes any change in structure (i.e., a qualitative change) of a protein. Such modifications can include, but are not limited to, changes in the amino acid sequence, transcriptional or translational splice variation, pre- or post- translational modifications to the DNA or RNA sequence, addition of macromolecules or small molecules to the DNA, RNA or protein, such as peptides, ions, vitamins, atoms, sugar-containing molecules, lipid-containing molecules, small molecules and the like, as well-known in the art. One type of protein modification is by one or more changes in the amino acid sequence (substitution, deletion or insertion). Such changes could include, at one or more amino acids, a change from a charged amino acid to a different charged amino acid, a non-charged to a charged amino acid, a charged amino acid to a non-charged amino acid as discussed, *infra. or supra.* Another type of protein modification is by changes in processing of the protein in the cell. A non-limiting example is where some proteins have an "address label" specifying where in (or outside of) the cell they should be used. Such a label or tag can be in the form of a peptide, a sugar or a lipid, which when added or removed from the protein, determines where the protein is located in the cell.

A further type of protein modification is due to the attachment of other macromolecules to a protein. This group can include, but is not limited to, any addition/removal of such a macromolecule. These molecules can be of many types and can be either permanent or temporary. Examples include: (i) polyribosylation, (ii) DNA/RNA (single or double stranded); (iii) lipids and phosphlipids (e.g., for membrane attachment); (iv) saccharides/polysaccharides; and (v) glycosylation (addition of different types of sugar and sialic acids -- in a variety of single and branched structures). Another type of protein modification is due to the attachment of other small molecules to proteins. Examples can include, but are not limited to: (i) phosphorylation; (ii) acetylation; (iii) uridylation; (iv) adenylation; (v) methylation, and (vi) capping (diverse complex modification of the N-terminus of the protein for assorted reasons). Most of these changes are often used to regulate a protein's activity. (v) and (vi) are also used to change the half-life of the protein itself. These protein changes can be detected on 2 dimensional gel electrophoresis incorporating several methods, such as labeling, changes in pI, antibodies or other specific techniques directed to the molecules themselves, as known in the art. Molecular weight changes can be, but may not usually be detectable by 2DGE. MALD (matrix assisted laser desorption of flight mass spectrometry) is preferred to detect and characterize these modifications. Such modifications are generally directed at improving upon any poor therapeutic characteristics of an unmodified LP-48 polypeptide by increasing that molecule's target specificity, solubility, stability, serum half-life, affinity for targeted receptors, susceptibility to proteolysis, resistance to clearing in vivo, ease of purification, and/or decreasing the antigenicity and/or required frequency of administration.

The term "cause" is intended to include the beginning molecular events that result in the symptoms of apoptosis, immunodeficiency, cancer, inflammation, and/or infectious diseases or are implicated in the organism's response to immunodeficiency, cancer, inflammation, and/or infectious disease; the term "mediate" includes any molecular events that form part of the causal chain of events that result in the symptoms of apoptosis, immunodeficiency, cancer, inflammation, and/or infectious disease or are part of the organism's response to immunodeficiency, cancer, inflammation, and/or infectious disease.

The term "HIS tag" where used herein refers to the LP-48 sequence, or portion thereof, fused to a highly rich histidine polypeptide sequence. The HIS tag has enough histidine residues to provide a unique purification means to select for the properties of the repeated histidine residues, yet is short enough such that it does not interfere with the activity of the extracellular domain sequence of LP-48.

Other exemplary tags include, but are not limited to, an "HA" tag, containing amino acids from a hemagglutinin fragment, and a "FLAG" tag (Immunex Corp., Seattle, WA), a six amino acid tag. These and other suitable tag polypeptides generally have at least six amino acid residues and usually between about 4 to about 20 amino acid residues (preferably, between about 4 to about 10 residues, and most preferably 6, such as HHHHHH). The HA tag corresponds to an epitope derived from the influenza hemagglutinin polypeptide (Wilson et al., Cell 37:767-778 (1984)). The fusion of the HA tag to the target polypeptide allows easy detection and recovery of the recombinant polypeptide with an antibody that recognizes the HA epitope. The FLAG tag is similarly applied for polypeptide detection and purification with an antibody that recognizes the FLAG epitope.

As used herein, the term "immunoadhesion," sometimes referred to as an Fc fusion, designates antibody-like molecules that combine the binding specificity of a heterologous protein (an "adhesion") with the effector functions of immunoglobulin constant domains. Structurally, the immunoadhesions comprise a fusion of an amino acid sequence with the desired binding specificity which is other than the antigen recognition and binding site of an antibody (i.e., is "heterologous"), and an immunoglobulin constant domain sequence. The adhesion part of an immunoadhesion molecule typically is a contiguous amino acid sequence comprising at least the binding site of a receptor or a ligand. The immunoglobulin constant domain sequence in the immunoadhesion may be obtained from any immunoglobulin, such as IgG-1, IgG-2, IgG-3 or IgG-4 subtypes, IgA (including IgA-1 and IgA-2), IgE, IgD or IgM.

The term "antagonist" is used in the broadest sense and includes any molecule that partially or fully blocks, inhibits, or neutralizes a biological activity of a native LP-48 polypeptide disclosed herein. In a similar manner, the term "agonist" is used in the broadest sense and includes any molecule that mimics a biological activity of a native LP-48 polypeptide disclosed herein. The term "antagonist" specifically includes antagonist antibodies or antibody fragments, ribozymes, anti-sense nucleic acids, as well as small organic molecules. Methods for identifying agonists or antagonists of an LP-48 polypeptide may comprise contacting an LP-48 polypeptide with a candidate agonist or antagonist molecule and measuring a detectable change in one or more biological activities normally associated with the LP-48 polypeptide.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules that lack antigen specificity. Polypeptides of the latter kind are, for example, produced at low levels by the lymph system and at increased levels by myelomas. The term "antibody" is used in the broadest sense and specifically covers, without limitation, intact monoclonal antibodies, polyclonal antibodies, multispecific antibodies (e.g., bispecific antibodies) formed from at least two intact antibodies, and antibody fragments so long as they exhibit the desired biological activity.

"Antibody fragments" comprise a portion of an intact antibody, preferably the antigen binding or variable region of the intact antibody. Examples of antibody fragments include Fab, Fab', F(ab')l and Fv fragments; diabodies; linear antibodies (Zapata et al., *Protein Engin. 8* (10): 1057-1062 (1995)); single-chain antibody molecules; and multispecific antibodies formed from antibody fragments.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domain, which enables the sFv to form the desired structure for antigen binding. For a review of sFv, see Pluckthun, *The Pharmacology of Monoclonal Antibodies,* vol. 113, Rosenburg and Moore, eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404.097, WO 93/11161; and Hollinger et al., *Proc. Natl. Acad. Sci. USA* 90: 6444-6448 (1993) .

An "isolated" antibody is one that has been identified and separated and/or recovered from a component of its natural environment. Contaminant components of its natural environment are materials that would interfere with diagnostic or therapeutic uses for the antibody, and may include enzymes, hormones, and other proteinaceous or non-proteinaceous solutes. It is preferred that the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue, or preferably, silver stain. Isolated antibody includes the antibody in situ within recombinant cells since at least one component of the antibody's natural environment will not be present. Ordinarily, however, isolated antibody will be prepared by at least one purification step.

The term "antibody" as used herein describes antibodies, fragments of antibodies (such as, but not limited, to Fab, Fab', F(ab')₂, and Fv fragments), and modified versions thereof, as well-known in the art (e.g., chimeric, humanized, recombinant, veneered, resurfaced or CDR-grafted). The term "antibody" is meant to include polyclonal antibodies, monoclonal antibodies (MAbs), chimeric antibodies, multispecific (e.g., bi-specific, trispecific, etc.) antibodies, single-chain polypeptide binding molecules, and anti-idiotypic (anti-id) antibodies.

The term "isolated" in reference to a LP-48 polynucleotide or polypeptide refers to a LP-48 molecule that has been identified and separated and/or recovered from at least one contaminant from which it has been produced. Contaminant components are materials that may include cellular components, such as enzymes, hormones, and other proteinaceous or non-proteinaceous solutes. Ordinarily, however, isolated LP-48 molecules will be prepared by at least one purification step.

"Conservative substitution" or "conservative amino acid substitution" refers to a replacement of one or more amino acid residue(s) in a protein or peptide. Conservative substitutions of interest are shown in Table 1 along with preferred substitutions. If such substitutions maintain or improve the desired function, then more substantial changes, then listed as exemplary substitutions in Table 1, or as further described below in reference to amino acid classes, are introduced and the products screened. A substitution at a particular position can constitute 1, 2, 3, 4, 5, 10, 15, 20 amino acids.

**Table 1**

| Conservative Substitutions | | |
|---|---|---|
| Original Residue | Example Substitutions | Preferred Substitutions |
| Ala | val, leu, ile | val |
| Arg | lys, gln, asn | lys |
| Asn | gln | gln |
| Asp | glu | glu |
| Cvs | ser | ser |
| Gln | asn | asn |
| Glu | asp | asp |
| Gly | pro, ala | ala |
| His | asn, gln, lys, arg | arg |
| Ile | leu, val, met, ala | leu |
| Leu | norleucine, ile, | ile |
| Lys | arg, gln, asn | arg |
| Met | leu, phe, ile | leu |
| Phe | leu, val, ile, ala, | leu |
| Pro | ala | ala |
| Ser | thr | thr |
| Thr | ser | ser |
| Trp | tyr,phe | tyr |
| Tyr | trp,phe,thr,ser | phe |
| Val | ile, leu, met, phe, | leu |

Naturally occurring residues are divided into groups based on common side-chain properties:
(1) hydrophobic: cys, ser, thr;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

The term "fragment thereof" as used herein refers to a fragment, piece, or sub-region of a nucleic acid or protein molecule whose sequence is disclosed herein, such that the fragment comprises 10, 15, 20 or more amino acids, or 15, 30, 45, 60 or more nucleotides that are contiguous in the parent protein or nucleic acid compound. When referring to a nucleic acid compound, "fragment thereof" refers to 20, 30, 45, 60 or more contiguous nucleotides, derived from the parent nucleic acid, and also, owing to the genetic code, to the complementary sequence. For example if the fragment entails the sequence 5'-AGCTAG-3', then "fragment thereof" would also include the complementary sequence, 3'-TCGATC-5'.

"Functional fragment" or "functionally equivalent fragment," as used herein, refers to a region, or fragment of a full length protein, or sequence of amino acids that are capable of competing with the endogenous or native LP-48 for binding to a natural or recombinantly expressed LP-48 receptor. The present invention also provides for the use of fragments of the LP-48 proteins disclosed herein wherein said fragments retain ability to bind a natural ligand. As used herein, "functional fragments" includes fragments whether or not fused to additional sequences that retain and exhibit, under appropriate conditions, measurable bioactivity, for example, protection against LPS challenge in vivo. Functional fragments of the proteins disclosed herein may be produced as described herein, preferably using cloning techniques to engineer smaller versions of the a functioning LP-48, lacking sequence from the 5' end, the 3' end, from both ends, or from an internal site.

The present invention includes methods of using LP-48 proteins as well as any related functional analogs that retain the ability to be employed therapeutically according to the present invention. Modifications of the amino acid sequence can generally be made in accordance with the substitutions provided in Table 1.

The term "homolog" refers to a molecule from a given organism that exhibits sequence similarity and/or identity and has substantially similar biological activity as a molecule from a different organism. For example, a mouse polypeptide that functions in the mouse in a manner equivalent to which human LP-48 functions in humans may be referred to as a mouse homolog of LP-48. The term "homolog" is also intended to encompass two or more genes or proteins from different organisms or within a single organism that exhibit sequence similarity and/or identity. The term "homolog" as used herein includes allelic variants, as well as splice variants from a LP-48 polynucleotide sequence.

As used herein, a "functional homolog" of LP-48 maintains the binding capability such that it is capable of binding at least one of its natural ligands. Further, the binding affinity typically will be at least 30%, 40%, or 50% of the native LP-48 of SEQ ID NO:2; and more preferably at least 60%, 70%, 80%, 90%, 95%, 100% or greater than 100%, 110%, 120%, 150%, 200% or 1000%.

The term "fusion protein" denotes a hybrid protein molecule not found in nature comprising a translational fusion or enzymatic fusion in which two or more different protein segments not naturally found in a contiguous sequence are covalently linked together, generally on a single peptide chain.

Also as used herein the term "abnormal apoptosis" refers to excessive and/or improper apoptosis. Typically abnormal apoptosis is observed in cells and tissues that have undergone physical, chemical or biological insult. Such insults include, but are not limited to, physical injury, viral infection, bacterial infection, ischemia, irradiation, chemotherapy, and the like.

The term "similar" or "similarity" describes the relationship between different nucleic acid compounds or amino acid sequences in which said sequences or molecules are related by partial sequence identity or sequence similarity at one or more blocks or regions within said molecules or sequences.

In referring to amino acid sequences, the term "similar" or "similarity" describes amino acid residues which are either identical between different amino acid sequences, or represent conservative amino acid substitutions between different sequences. Conservative amino acid substitutions are listed in Table 1 and discussed infra. The term "identity" describes amino acid residues which are identical between different amino acid sequences. Amino acid sequence similarity or identity with respect to the LP-48 amino acid sequence identified herein is defined as the percentage of amino acid residues in a candidate sequence that are similar or identical with the amino acid residues in an LP polypeptide sequence, after aligning the sequences and introducing gaps, if necessary, to achieve the maximum percent sequence similarity or identity.

Alignment for purposes of determining percent amino acid sequence identity can be achieved in various ways that are within the skill in the art, for instance, using publicly available computer software such as ALIGN, ALIGN-2, Megalign (DNASTAR) or BLAST (e.g., Blast, Blast-2, WU-Blast-2) software. Those skilled in the art can determine appropriate parameters for measuring alignment, including any algorithms needed to achieve maximal alignment over the full length of the sequences being compared. For example, in the generation of percent identity values generated using WU-BLAST-2 [Altschul, *et al., Methods in Enzymology* 266:460-80 (1996)], the WU-BLAST-2 search parameters are typically set to the default values. Those not set to default values, i.e., the adjustable parameters, are typically set with the following values: overlap span = 1; overlap fraction = 0.125; word threshold (T) = 11; and scoring matrix = BLOSUM 62. For purposes herein, a percent amino acid sequence identity value is determined by dividing (a) the number of matching identical amino acid residues between the amino acid sequence of the LP polypeptide of interest and the comparison amino acid sequence of interest (i.e., the sequence against which the LP polypeptide of interest is being compared) as determined by WU-BLAST-2, by (b) the total number of amino acid residues of the LP polypeptide of interest, respectively.

The term "identity" with respect to nucleic acid sequences as used herein is defined as the percentage of nucleotides in a candidate sequence that are identical with the nucleotides in a test sequence after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as ALIGN, Align-2, Megalign (DNASTAR), or BLAST (e.g., Blast, Blast-2) software. Those skilled in the art can determine appropriate parameters for measuring alignment, including any algorithms needed to achieve maximal alignment over the full length of the sequences being compared. For purposes herein, however, percentage nucleic acid identity values are generated using the WU-BLAST-2 (BlastN module) program [Altschul, *et al*., *Methods in Enzymology* 266:460-80 (1996)]. Most of the WU-BLAST-2 search parameters are set to the default values. Those not set default values, i.e., the adjustable parameters, are set with the following values: overlap span = 1; overlap fraction = 0.125; word threshold (T) = 11; and scoring matrix = BLOSUM62. For purposes herein, a percent nucleic acid sequence identity value is determined by dividing (a) the number of matching identical nucleotides between the nucleic acid sequence of the LP polypeptide-encoding nucleic acid molecule of interest and the comparison nucleic acid molecule of interest (i.e., the sequence against which the LP polypeptide-encoding nucleic acid molecule of interest is being compared) as determined by WU-BLAST-2, by (b) the total number of nucleotides of the LP polypeptide-encoding nucleic acid molecule of interest.

In addition to calculating percent sequence identity, the BLAST algorithm also performs a statistical analysis of the similarity between two sequences (see, e.g., Karlin and Altschul, 1993, Proc. Nat'l Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

The term "host cell" as used herein refers to any eukaryotic or prokaryotic cell that is suitable for propagating and/or expressing a cloned gene contained on a vector that is introduced into said host cell by, for example, transformation or transfection, or the like.

The term "hybridization" as used herein refers to a process in which a single-stranded nucleic acid compound (or probe) joins with a complementary strand through nucleotide base pairing. The degree of hybridization depends upon, for example, the degree of similarity, the stringency of hybridization, and the length of hybridizing strands. "Selective hybridization" refers to hybridization under conditions of high stringency.

"Stringency" of hybridization reactions is readily determinable by one of ordinary skill in the art, and generally is an empirical calculation dependent upon probe length, washing temperature, and salt concentration. In general, longer nucleic acid probes required higher temperatures for proper annealing, while shorter nucleic acid probes need lower temperatures. Hybridization generally depends on the ability of denatured DNA to reanneal when complementary strands are present in an environment below their melting temperature. The higher the degree of desired homology between the probe and hybridizable sequence, the higher the relative temperature that can be used. As a result, it follows that higher relative temperatures would tend to make the reactions more stringent, while lower temperatures less so. For additional details and explanation of stringency of hybridization reactions, see Ausubel, *et al., Current Protocols in Molecular Biology,* Wiley Interscience Publishers (1995).

"Stringent conditions" or "high stringency conditions," as defined herein, may be identified by those that
(1) employ low ionic strength and high temperature for washing, for example, 15 mM sodium chloride/1.5 mM sodium citrate/0.1% sodium dodecyl sulfate at 50 degrees C;
(2) employ during hybridization a denaturing agent, such as formamide, for example, 50% (v/v) formamide with 0.1% bovine serum albumin/0.1% ficoll/0.1% polyvinylpyrrolidone/50 mM sodium phosphate buffer at pH 6.5 with 750 mM sodium chloride/75 mM sodium citrate at 42 degrees C; or (3) employ 50% formamide, 5X SSC (750 mM sodium chloride, 75 mM sodium citrate), 50 mM sodium phosphate (pH 6.8), 0.1% sodium pyrophosphate, 5X Denhardt's solution, sonicated salmon sperm DNA (50 µg/mL), 0.1% SDS, and 10% dextran sulfate at 42 degrees C with washes at 42 degrees C in 0.2X SSC (30 mM sodium chloride/3 mM sodium citrate) and 50% formamide at 55 degrees C, followed by a high-stringency wash consisting of 0.1X SSC containing EDTA at 55 degrees C.

"Moderately stringent conditions" may be identified as described by Sambrook, *et al.* [*Molecular Cloning: A Laboratory Manual,* New York: Cold Spring Harbor Press, (1989)], and include the use of washing solution and hybridization conditions (e.g., temperature, ionic strength and %SDS) less stringent than those described above. An example of moderately stringent conditions is overnight incubation at 37 degrees C in a solution comprising: 20% formamide, 5X SSC (750 mM sodium chloride, 75 mM sodium citrate), 50 mM sodium phosphate at pH 7.6, 5X Denhardt's solution, 10% dextran sulfate, and 20 mg/mL denatured sheared salmon sperm DNA, followed by washing the filters in 1X SSC at about 37 to 50 degrees C. The skilled artisan will recognize how to adjust the temperature, ionic strength, etc., as necessary to accommodate factors such as probe length and the like.

"Isolated nucleic acid compound" refers to any RNA or DNA sequence, however constructed or synthesized, which is locationally distinct from its natural location.

The term "mature protein" or "mature polypeptide" as used herein refers to the form(s) of the protein produced by expression in a mammalian cell. It is generally hypothesized that once export of a growing protein chain across the rough endoplasmic reticulum has been initiated, proteins secreted by mammalian cells have a signal sequence which is cleaved from the complete polypeptide to produce a "mature" form of the protein. Oftentimes, cleavage of a secreted protein is not uniform and may result in more than one species of mature protein. The cleavage site of a secreted protein is determined by the primary amino acid sequence of the complete protein and generally cannot be predicted with complete accuracy. As identified in SEQ ID NO:2, the native signal peptide of LP-48 is encoded from about amino position 1 (methionine-1) to about alanine-18. It should be noted, however, the C-terminal boundary of the signal peptides may vary, but likely by no more than about 5 amino acids, preferably no more than about 4 amino acids, more preferably no more than about 3 amino acids, more preferably no more than about 2 amino acids, more preferably by no more than about 1 amino acid. Cleavage sites for a secreted protein may also be determined experimentally by amino-terminal sequencing of the one or more species of mature proteins found within a purified preparation of the protein.

A "nucleic acid probe" or "probe" as used herein is a labeled nucleic acid compound that hybridizes with another nucleic acid compound. "Nucleic acid probe" means a single stranded nucleic acid sequence that will combine with a complementary or partially complementary single stranded target nucleic acid sequence to form a double-stranded molecule. A nucleic acid probe may be an oligonucleotide or a nucleotide polymer. A probe will usually contain a detectable moiety that may be attached to the end(s) of the probe or be internal to the sequence of the probe.

The term "plasmid" refers to an extrachromosomal genetic element. The plasmids disclosed herein are commercially available, publicly available on an unrestricted basis, or can be constructed from readily available plasmids in accordance with published procedures.

A "primer" is a nucleic acid fragment which functions as an initiating substrate for enzymatic or synthetic elongation of, for example, a nucleic acid compound.

The term "promoter" refers to a nucleic acid sequence that directs transcription, for example, of DNA to RNA. An inducible promoter is one that is regulatable by environmental signals, such as carbon source, heat, or metal ions, for example. A constitutive promoter generally operates at a constant level and is not regulatable.

"Recombinant DNA cloning vector" as used herein refers to any autonomously replicating agent, including, but not limited to, plasmids and phages, comprising a DNA molecule into which one or more additional DNA segments can be or have been incorporated.

The term "recombinant DNA expression vector" or "expression vector" as used herein refers to any recombinant DNA cloning vector (such as a plasmid or phage), in which a promoter and other regulatory elements are present, thereby enabling transcription of an inserted DNA, which may encode a polypeptide.

"Substantially pure" used in reference to a LP-48 polynucleotide or polypeptide means that said "LP-48" is separated from other cellular and non-cellular molecules, including other proteins, lipids, carbohydrates or other materials with which it is naturally associated when produced recombinantly or synthesized without any general purifying steps. A "substantially pure" LP-48 polypeptides described herein could be prepared by a variety of techniques well known to the skilled artisan, including, for example, the described methods of LP-48 purification referred to or described herein. In preferred embodiments, the LP-48 polypeptide will be purified (1) to greater than 95% by weight of LP-48 polypeptide to the weight of total protein as determined by the Lowry method, and most preferably more than 99% by weight to the weight of total protein, (2) to a degree sufficient to obtain at least 15 residues of N-terminal or internal amino acid sequence by use of a spinning cup sequenator, or (3) to apparent homogeneity by SDS-PAGE under reducing or nonreducing conditions using Coomassie Blue, or preferably, silver stain, such that the major band constitutes at least 95%, and more preferably 99%, of the stained protein observed on the gel.

The term "sufficient similarity" refers to a first amino acid or nucleotide sequence which contains a sufficient or minimum number of identical or related amino acid substitutions (for related amino acids see Table 1 for conservative substitutions and discussion infra. of groups) or nucleotides to a second amino acid or nucleotide sequence such that the first and second amino acid or nucleotide sequences have a common structural domain and/or common functionality. For example, amino acid or nucleotide sequences which have at least about 85% identity, more preferably at least about 90% identity, more preferably at least about 95% identity, more preferably at least about 99% identity, and most preferably 100% identity.

The term "vector" as used herein refers to a nucleic acid compound used for introducing exogenous or endogenous DNA into host cells. A vector comprises a nucleotide sequence that may encode one or more protein molecules. Plasmids, cosmids, viruses, and bacteriophages, in the natural state or which have undergone recombinant engineering, are examples of commonly used vectors.

The term "down-regulate" as used herein includes to reduce, inhibit, or otherwise cause a decrease in the level or activity of a molecule(s) or a process. For example, LP-48 polypeptide may be used to down-regulate the level or activity of a cytokine in cells, such that the level or activity of the cytokine is decreased compared to the level or activity that would be present in the absence of the LP-48 polypeptide. Alternatively, LP-48 polypeptide may be used to down-regulate a process, such as inflammation.

The terms "treating", "treatment" and "therapy" as used herein refer to curative therapy, prophylactic therapy, and preventative therapy. An example of "preventative therapy" is the prevention or lessening of a targeted disease or related condition thereto. Those in need of treatment include those already with the disease or condition as well as those prone to have the disease or condition those in whom the disease or condition is to be prevented. The terms "treating", "treatment", and "therapy" as used herein also describe the management and care of a patient for the purpose of combating a disease, or related condition, and includes the administration of LP-48 to alleviate the symptoms or complications of said disease, condition.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

The term "patient" as used herein refers to any mammal, including humans, domestic and farm animals, and zoo, sports or pet animals, such as cattle (e.g. cows), horses, dogs, sheep, pigs, rabbits, goats, cats, and non-domesticated animals like mice and rats. In a preferred embodiment of the invention, the mammal is a human or mouse.

Administration "in combination with" one or more further therapeutic agents includes simultaneous (concurrent) and consecutive administration in any order.

A "therapeutically-effective amount" is the minimal amount of an LP-48 polypeptide or LP-48 recognizing antibody that is necessary to impart therapeutic benefit or desired biological effect to a patient. For example, a "therapeutically-effective amount" of a LP-48 polypeptide to a patient suffering or prone to suffer from an LP-48 associated disease or condition is such an amount which induces, ameliorates or otherwise causes an improvement in the pathological symptoms, disease progression, physiological conditions associated with, or resistance to succumbing to a disorder principally characterized by immunodeficiency, cancer, inflammation, and/or infectious disease when the LP-48 polypeptide and/or LP-48 epitope recognizing antibody is administered. The precise amount of LP-48 polypeptide or LP-48 epitope recognizing antibody administered to a particular patient will depend upon numerous factors, e.g., such as the specific binding activity of the molecule, the delivery device employed, physical characteristics, its intended use, and patient considerations, can readily be determined by one skilled in the art, based upon the information provided herein and that which is known in the art.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecule weight (less than about 10 residues) polypeptides; proteins, such as serum albumin, gelatin, or immunoglobulins; hydrophilic polymers such as polyvinylpyrrolidone; amino acids such as glycine. glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN^{®}, polyethylene glycol (PEG), and PLURONICS^{™}.

"Pharmaceutically acceptable salt" includes, but is not limited to, salts prepared with inorganic acids, such as chloride, sulfate, phosphate, diphosphate, hydrobromide, and nitrate salts, or salts prepared with an organic acid, such as malate, maleate, fumarate, tartrate, succinate, ethylsuccinate, citrate, acetate, lactate, methanesulfonate, benzoate, ascorbate, para-toluenesulfonate, palmoate, salicylate and stearate, as well as estolate, gluceptate and lactobionate salts. Similarly, salts containing pharmaceutically acceptable cations include, but are not limited to, sodium, potassium, calcium, aluminum, lithium, and ammonium (including substituted ammonium).

The term "symptom" in reference to sepsis, gram negative bacteremia, allergic responses, allergic autoimmune diseases, type 1 diabetes, Th1-dependent insulitis, immunodeficiencies, cancers, inflammation, infectious diseases is meant to include, but not limited to, one or more of the following: chills, profuse sweating, fever, weakness, hypotension, leukopenia, intravascular coagulation, shock, respiratory distress, organ failure, prostration, ruffled fur, diarrhea, eye exudate, and death, alone or in combination. This list is not meant to be exclusive, but may be supplemented with symptoms or combinations of symptoms that a person of ordinary skill would recognize as associated with sepsis, gram negative bacteremia, allergic responses, allergic autoimmune diseases, type 1 diabetes, Th1-dependent insulitis, immunodeficiencies, cancers, inflammation, infectious diseases. A symptom associated with sepsis, gram negative bacteremia, allergic responses, allergic autoimmune diseases, type 1 diabetes, Th1-dependent insulitis, immunodeficiencies, cancers, inflammation, infectious diseases may also be associated with another condition.

### Protein Production

Skilled artisans will recognize that the LP-48 polypeptides utilized in embodiments of the present invention can be synthesized by a number of different methods, such as chemical methods well known in the art, including solid phase peptide synthesis or recombinant methods. Both methods are described in U.S. Patent 4,617,149.

The principles of solid phase chemical synthesis of polypeptides are well known in the art and may be found in general texts in the area. See, e.g., H. Dugas and C. Penney, Bioorganic Chemistry (1981) Springer-Verlag, New York, 54-92. For example, peptides may be synthesized by solid-phase methodology utilizing an Applied Biosystems 430A peptide synthesizer (Applied Biosystems, Foster City, CA) and synthesis cycles supplied by Applied Biosystems.

The proteins utilized in the present invention can also be produced by recombinant DNA methods using the LP-48 polynucleotide sequences described herein. Recombinant methods are preferred if a high yield is desired. Expression of the LP-48 can be carried out in a variety of suitable host cells, well known to those skilled in the art. For this purpose, the LP-48 constructs are introduced into a host cell by any suitable means, well known to those skilled in the art. Chromosomal integration of LP-48 expression vectors may be used as well as suitable extra-chromosomally maintained expression vectors so that the coding region of the LP-48 is operably-linked to a constitutive or inducible promoter.

The basic steps in the recombinant production of the LP-48 protein are:
a) constructing a recombinant, synthetic or semi-synthetic DNA encoding LP-48 protein;
b) integrating said DNA into an expression vector in a manner suitable for expressing the LP-48 protein;
c) transforming or otherwise introducing said vector into an appropriate eukaryotic or prokaryotic host cell forming a recombinant host cell;
d) culturing said recombinant host cell in a manner to express the LP-48 protein; and
e) recovering and substantially purifying the LP-48 protein by any suitable means well known to those skilled in the art.

Production of LP-48 products also include routes where direct chemical synthetic procedures are employed as well as products produced by recombinant techniques from a eukaryotic host, including, for example, yeast, higher plant, insect and mammalian cells. Depending upon the host employed in a recombinant production procedure, the polypeptides of the present invention can be glycosylated or can be non-glycosylated. Additionally, the amino acid sequence of a LP-48 may optionally include a conservative substitution. Preferred LP-48 molecules are glycosylated as would occur in eukaryotic hosts. In addition, the LP-48 polypeptides can also include an initial modified methionine residue, in some cases as a result of host-mediated processes. Such methods are described in many standard laboratory manuals, such as Sambrook, supra, Chapters 17.37-17.42; Ausubel, supra, Chapters 10, 12, 13, 16, 18 and 20.

Those skilled in the art will recognize that owing to the degeneracy of the genetic code (i.e., 64 codons which encode 20 amino acids), numerous "silent" substitutions of nucleotide base pairs could be introduced into a LP-48 polynucleotide sequence without altering the identity of the encoded amino acid(s) or protein product.

Fragments of the proteins disclosed herein may be generated by any number of suitable techniques, including chemical synthesis. For instance constant regions of immunoglobulins can be obtained by papain digestion of antibodies. Such proteolytic digestion of, for example, SEQ ID NO:2 can produce the Ig constant region which then can be covalently linked to the extracellular domain of LP-48. Alternatively, recombinant DNA mutagenesis techniques can provide LP-48 molecules (see generally. e.g. K. Struhl, "Reverse biochemistry: Methods and applications for synthesizing yeast proteins in vitro," Meth. Enzymol. 194:520-535). For example, a nested set of deletion mutations are introduced into a LP-48 encoding polynucleotide such that varying amounts of the protein coding region are deleted, either from the amino terminal end, or from the carboxyl end of the protein molecule. Further, additional changes or additions to the molecule can be made. This method can also be used to create internal fragments of the intact protein in which both the carboxyl and/or amino terminal ends are removed. Several appropriate nucleases can be used to create such deletions, for example Ba131, or in the case of a single stranded nucleic acid compound, mung bean nuclease. For simplicity, it is preferred that the intact LP-48 gene be cloned into a single-stranded cloning vector, such as bacteriophage M13, or equivalent. If desired, the resulting gene deletion fragments can be subcloned into any suitable vector for propagation and expression of said fragments in any suitable host cell.

LP-48 can additionally be fused to a marker protein or an epitope tag. Such fusions include, but are not limited to, fusions to an enzyme, fluorescent protein, or luminescent protein which provide a marker function; or fusions to any amino acid sequence which can be employed for purification of the polypeptide or a proprotein sequence.

Methods of constructing fusion proteins (chimeras) composed of the binding domain of one protein and the constant region of an immunoglobulin (herein designated as "LP-48-Ig") are generally known in the art. For example, chimeras containing the Fc region of human IgG and the binding region of other protein receptors are known in the art for chimeric antibodies. LP-48-Ig structures of the present invention can be constructed using methods similar to the construction of chimeric antibodies. In chimeric antibody construction the variable domain of one antibody of one species is substituted for the variable domain of another species (see EP 0 125 023; EP 173,494; Munro et al., Nature, 312:597 (1984); Neuberger et al. Nature, 312:604-608 (1984); Sharon et al., Nature, 309:364-367; Morrison et al., Ann. Rev. Immunol., 2:239-256 (1984); Morrison et al., 1985; Boulianne et al., Nature, 312:643-646 (1984); Capon et al., Nature, 337:525-531 (1989); Traunecker et al., Nature, 339:68-70 (1989)). Here, a functional domain of the LP-48 polypeptide is substituted for the variable domain of the recipient antibody structure.

Generally, methods for constructing LP-48 fusion proteins include use of recombinant DNA technology. The DNA encoding a functional domain can optionally be fused with additional domains or segments of the LP-48 polypeptide or with an Ig constant region. A polynucleotide encoding any domain of a LP-48 polypeptide can be obtained by PCR or by restriction enzyme cleavage. This DNA fragment is readily inserted proximal to DNA encoding an immunoglobulin light or heavy chain constant region and, if necessary, the resulting construct is tailored by mutagenesis, to insert, delete, or change the codon sequence. Preferably, the selected immunoglobulin region is a human immunoglobulin region when the chimeric molecule is intended for in vivo therapy for humans. Most preferably, the selected immunoglobulin region is an IgG region. DNA encoding immunoglobulin light or heavy chain constant regions are known or readily available from cDNA libraries or can be synthesized (see for example Adams et al., Biochemistry, 19:2711-2719 (1980); Gough et al., Biochemistry, 19:2702-2710 (1980); Dolby et al., 1980; Rice et al., Proc. Nat'1. Acad. Sci., 79:7862-7865 (1982); Falkner et al., Nature, 298:286-288 (1982); and Morrison et al., Ann. Rev. Immunol., 2:239-256 (1984)). Other teachings of preparing chimeric molecules are known from the preparation of immunoadhesion chimeras, such as CD4-Ig (Capon et al., Nature, 337:525-531 (1989); Byrn et al., Nature, 344:667 (1990)) and TNFR chimeras, such as TNFR-IgG (Ashkenazi, et al., Proc. Natl. Acad. Sci., 88:10535-10539 (1991); Peppel et al., J. Cell. Biochem. Supp. 15F-P439:118 (1991)).

### Protein Purification

Generally, LP-48 polypeptides are produced recombinantly. Once expressed they can be isolated from the cells by applying standard protein isolation techniques to the lysates or purified from the media. The monitoring of the purification process can be accomplished by using standard Western blot techniques or radioimmunoassays or other standard immunoassay techniques.

LP-48 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, size exclusion chromatography, and lectin chromatography. Preferably, high performance liquid chromatography ("HPLC"), cation exchange chromatography, affinity chromatography, size exclusion chromatography, or combinations thereof, are employed for purification. Particular methods of using protein A or G chromatography for purification are known in the art and are particularly applicable where the LP-48 contains immunoglobulin Fc region. Protein A and G binds the Fc regions of IgG antibodies, and therefore makes a convenient tool for the purification of LP-48 polypeptides containing the IgG region. LP-48 polypeptide purification is meant to include purified parts of the chimeric (the extracellular region and the immunoglobulin constant region) that are purified separately and then combined by disulfide bonding, cross-linking or the like.

The purification of LP-48 polypeptides can be accomplished by a number of special techniques known in the art (Kwon et al., J. Biol. Chem., 272:14272-14276 (1997); Kwon et al., Cell, 272:14272-14276 (1997); Emery, J. G., et al., J. Biol. Chem., 273:14363-14367 (1998); Harrop et al., J. Biol. Chem., 273(42):27548-27556 (1998); Harrop et al., J. Immunol., 161:1786-1794 (1998)) that take particular advantage of structural and functional features of these molecules. Further, a number of advantageous protein sequences can be incorporated into the LP-48 polypeptide produced, such as factor Xa cleavage sites or a HIS tag sequence or the incorporation of specific epitopes, as is known in the art.

### Expressing Recombinant LP-48 Proteins in Host Cells

Prokaryotes may be employed in the production of recombinant LP-48 proteins. For example, the Escherichia coli K12 strain 294 (ATCC No. 31446) is particularly useful for the prokaryotic expression of foreign proteins. Other strains of E. coli, bacilli such as Bacillus subtilis, enterobacteriaceae such as Salmonella typhimurium or Serratia marcescans, various Pseudomonas species and other bacteria, such as Streptomyces, may also be employed as host cells in the cloning and expression of the recombinant proteins of this invention.

Promoter sequences suitable for driving the expression of genes in prokaryotes include β-lactamase (e.g. vector pGX2907, ATCC 39344, contains a replicon and β-lactamase gene), lactose systems (Chang et al., Nature (London), 275:615 (1978); Goeddel et al., Nature (London), 281:544 (1979)), alkaline phosphatase, and the tryptophan (trp) promoter system (vector pATH1 (ATCC 37695)), which is designed to facilitate expression of an open reading frame as a trpE fusion protein under the control of the trp promoter. Hybrid promoters such as the tac promoter (isolatable from plasmid pDR540, ATCC-37282) are also suitable. Still other bacterial promoters, whose nucleotide sequences are generally known, may be ligated to DNA encoding the protein of the instant invention, using linkers or adapters to supply any required restriction sites. Promoters for use in bacterial systems also will contain a Shine-Dalgarno sequence operably linked to the DNA encoding the desired polypeptides. These examples are illustrative rather than limiting.

The LP-48 proteins required to practice the present invention may be synthesized either by direct expression or as a fusion protein comprising the protein of interest as a translational fusion with another protein or peptide that may be removed by enzymatic or chemical cleavage. It is often observed in the production of certain peptides in recombinant systems that expression as containing other desired sequences prolongs the lifespan, increases the yield of the desired peptide, or provides a convenient means of isolating the protein. This is particularly relevant when expressing mammalian proteins in prokaryotic hosts. A variety of peptidases (e.g., enterokinase and thrombin) which cleave a polypeptide at specific sites or digest the peptides from the amino or carboxy termini (e.g., diaminopeptidase) of the peptide chain are known. Furthermore, particular chemicals (e.g., cyanogen bromide) will cleave a polypeptide chain at specific sites. The skilled artisan will appreciate the modifications necessary to the amino acid sequence (and synthetic or semi-synthetic coding sequence if recombinant means are employed) to incorporate site-specific internal cleavage sites. See e.g., P. Carter, "Site Specific Proteolysis of Fusion Proteins", Chapter 13, in Protein Purification: From Molecular Mechanisms to Large Scale Processes, American Chemical Society, Washington, D.C. (1990).

In addition to prokaryotes, a variety of amphibian expression systems such as frog oocytes, and mammalian cell systems can be used. The choice of a particular host cell depends to some extent on the particular expression vector used. Exemplary mammalian host cells include 293 (e.g., ATCC CCL 1573), HepG-2 (ATCC HB 8065), CV-1 (ATCC CCL 70), LC-MK2 (ATCC CCL 7.1), 3T3 (ATCC CCL 92), CHO-K1 (ATCC CCL 61), HeLa (ATCC CCL 2), RPMI8226 (ATCC CCL 155), H4IIEC3 (ATCC CCL 1600), C127I (ATCC CCL 1616), HS-Sultan (ATCC CCL 1484), and BHK-21 (ATCC CCL 10), for example.

A wide variety of vectors are suitable for transforming mammalian host cells. For example, the pSV2-type vectors comprise segments of the simian virus 40 (SV40) genome required for transcription and polyadenylation. A large number of plasmid pSV2-type vectors have been constructed, such as pSV2-gpt, pSV2-neo, pSV2-dhfr, pSV2-hyg, and pSV2-β-globin, in which the SV40 promoter drives transcription of an inserted gene. These vectors are widely available from sources such as the American Type Culture Collection (ATCC), 12301 Parklawn Drive, Rockville, Maryland, 20852, or the National Center for Agricultural Utilization Research, 1815 North University Street, Peoria, Illinois 61604-39999.

Promoters suitable for expression in mammalian cells include the SV40 late promoter, promoters from eukaryotic genes, such as, for example, the estrogen-inducible chicken ovalbumin gene, the interferon genes, the glucocorticoid-inducible tyrosine aminotransferase gene, the thymidine kinase gene promoter, and the promoters of the major early and late adenovirus genes.

Plasmid pRSVcat (ATCC 37152) comprises portions of a long terminal repeat of the Rous Sarcoma virus, a virus known to infect chickens and other host cells. This long terminal repeat contains a promoter that is suitable for this use. (H. Gorman et al., Proc. Nat. Acad. Sci. (USA), 79, 6777 (1982)). The plasmid pMSVi (NRRL B-15929) comprises the long terminal repeats of the Murine Sarcoma virus, a virus known to infect mouse and other host cells. The mouse metallothionein promoter has also been well characterized for use in eukaryotic host cells. This promoter is present in the plasmid pdBPV-MMTneo (ATCC 37224) which can serve as the starting material for the construction of other expression plasmids that would also be useful in producing LP-48 polypeptides.

Transfection of mammalian cells with vectors can be performed by a plurality of well known processes including, but not limited to, protoplast fusion, calcium phosphate coprecipitation, electroporation and the like. See, e.g., Sambrook, et al., Molecular Cloning: A Laboratory Manual, 2^{nd} Edition, Cold Spring Harbor, N.Y. (1989).

Some viruses also make appropriate vectors. Examples include the adenoviruses, the adeno-associated viruses, the vaccinia virus, the herpes viruses, the baculoviruses, and the Rous Sarcoma virus, as described in U.S. Patent 4,775,624.

Eukaryotic microorganisms such as yeast and other fungi are also suitable host cells. The yeast Saccharomyces cerevisiae is the preferred eukaryotic microorganism. Other yeasts such as Kluyveromyces lactis and Pichia pastoris are also suitable. For expression in Saccharomyces, the plasmid YRp7 (ATCC-40053), for example, may be used. See, e.g., L. Stinchcomb et al., Nature, 282, 39 (1979); J. Kingsman et al., Gene, 7, 141 (1979); S. Tschemper et al., Gene, 10, 157 (1980). Plasmid YRp7 contains the TRP1 gene that provides a selectable marker for use in a trp1 auxotrophic mutant. Production of Antibodies

LP-48 epitope recognizing antibodies may be used in the treatment of various conditions relating to allergic responses, allergic autoimmune diseases, type 1 diabetes, Th1-dependent insulitis, immunodeficiencies, cancers, inflammation, infectious diseases. The production of antibodies, including both monoclonal and polyclonal, in animals, especially mice, is well known in the art. See, e.g., C. Milstein, Handbook of Experimental Immunology, (Blackwell Scientific Pub., 1986); J. Goding, Monoclonal Antibodies: Principles and Practice, (Academic Press, 1983). For the production of monoclonal antibodies the basic process begins with injecting a mouse, or other suitable animal, with an immunogen. The mouse is subsequently sacrificed and cells taken from its spleen are fused with myeloma cells, resulting in a hybridoma that reproduces in vitro. The population of hybridomas is screened to isolate individual clones, each of which secretes a single antibody species, specific for the immunogen. Each antibody obtained in this way is the clonal product of a single B cell.

Chimeric antibodies are described in U.S. Patent No. 4,816,567. This reference discloses methods and vectors for the preparation of chimeric antibodies. An alternative approach is provided in U.S. Patent No. 4,816,397. This patent teaches co-expression of the heavy and light chains of an antibody in the same host cell.

The approach of U.S. Patent 4,816,397 has been further refined in European Patent Publication No. 0 239 400. The teachings of this European patent publication are a preferred format for genetic engineering of monoclonal antibodies. In this technology the complementarily determining regions (CDRs) of a human antibody are replaced with the CDRs of a murine monoclonal antibody, thereby converting the specificity of the human antibody to the specificity of a murine antibody.

Single chain antibodies and libraries thereof are yet another variety of genetically engineered antibody technology that is well known in the art. (See, e.g, R.E. Bird, et al., Science 242:423-426 (1988); PCT Publication Nos. WO 88/01649, WO 90/14430, and WO 91/10737). Single chain antibody technology involves covalently joining the binding regions of heavy and light chains to generate a single polypeptide chain. The binding specificity of the intact antibody molecule is thereby reproduced on a single polypeptide chain.

The proteins or suitable fragments thereof required to generate polyclonal or monoclonal antibodies, and various inter-species hybrids, or humanized antibodies, or antibody fragments, or single-chain antibodies are disclosed herein. The techniques for producing antibodies are well known to skilled artisans. (See e.g., A.M. Campbell, Monoclonal Antibody Technology: Laboratory Techniques in Biochemistry and Molecular Biology, Elsevier Science Publishers, Amsterdam (1984); Kohler and Milstein, Nature 256, 495-497 (1975); Monoclonal Antibodies: Principles & Applications Ed. J.R.Birch & E.S. Lennox, Wiley-Liss (1995)).

The most preferred method of generating MAbs to the polypeptides and glycopeptides of the present invention comprises producing said MAbs in a transgenic mammal modified in such a way that they are capable of producing fully humanized MAbs upon antigenic challenge. Humanized MAbs and methods for their production are generally known in the art (see, e.g., U.S. Pat. Nos. 4,704,362, 4,816,567, 5,434,340, 5,545,806, 5,530,101, 5,569,825, 5,585,089, 5,625,126, 5,633,425, 5,643,763, 5,693,761, 5,693,762, 5,714,350, 5,874,299, 5,877,397, 5,939,598, 6,023,010, 6,054297, PCT/WO9634096, PCT/WO9633735, and PCT/WO9824893).

A protein used as an immunogen may be modified or administered in an adjuvant, by subcutaneous or intraperitoneal injection into, for example, a mouse or a rabbit. For the production of monoclonal antibodies, spleen cells from immunized animals are removed, fused with myeloma cells, such as SP2/0-Ag14 cells, and allowed to become monoclonal antibody producing hybridoma cells in the manner known to the skilled artisan. Hybridomas that secrete a desired antibody molecule can be screened by a variety of well known methods, for example ELISA assay, western blot analysis, or radioimmunoassay (Lutz et al., Exp. Cell Res. 175, 109-124 (1988); Monoclonal Antibodies: Principles & Applications Ed. J.R.Birch & E.S. Lennox, Wiley-Liss (1995)).

### Nucleic acids

The synthesis of the LP-48 polynucleotides (such as provided in SEQ ID NO:1) and related nucleic acids that would encode LP-48 polypeptides as defined herein or fragments thereof is well known in the art. See, e.g., E.L. Brown, R. Belagaje, M.J. Ryan, and H.G. Khorana, Methods in Enzymology, 68:109-151 (1979). Fragments of the DNA sequence corresponding to LP-48 sequences could be generated using a conventional DNA synthesizing apparatus, such as the Applied Biosystems Model 380A or 380B DNA synthesizers (Applied Biosystems, Inc., 850 Lincoln Center Drive, Foster City, CA 94404) using phosphoramidite chemistry, thereafter ligating the fragments so as to reconstitute the entire LP-48 sequence. Alternatively, phosphotriester chemistry may be employed to synthesize LP-48 nucleic acids. (See, e.g., M.J. Gait, ed., Oligonucleotide Synthesis, A Practical Approach, (1984)).

In an alternative methodology, namely PCR, the DNA sequences disclosed and described herein, comprising, for example, a portion or all of SEQ ID NO:1, can be produced from a plurality of starting materials. For example, starting with a cDNA preparation (e.g., cDNA library) derived from a tissue that expresses the LP-48 gene, suitable oligonucleotide primers complementary to regions of SEQ ID NO:1 or to any sub-region therein, are prepared as described in U.S. Patent No. 4,889,818. Other suitable protocols for the PCR are disclosed in PCR Protocols: A Guide to Method and Applications, Ed. Michael A. Innis et al., Academic Press, Inc. (1990). Using PCR, any region of the LP-48 gene can be targeted for amplification such that full or partial length gene sequences containing a functional extracellular domain may be produced.

It may be advantageous to use oligonucleotide primers. The sequence of such primers is designed using a LP-48 polynucleotide for use in detecting, amplifying, or mutating a defined segment of a gene or polynucleotide that encodes a LP-48 polypeptide using PCR technology.

The ionic strength and incubation temperature under which a probe will be used should also be taken into account. It is known that hybridization will increase as the ionic strength of the reaction mixture increases, and that the thermal stability of molecular hybrids will increase with increasing ionic strength. On the other hand, chemical reagents such as formamide, urea, DMSO and alcohols, which disrupt hydrogen bonds, increase the stringency of hybridization. Destabilization of hydrogen bonds by such reagents can greatly reduce the Tm (melting temperature). In general, optimal hybridization for synthetic oligonucleotide probes of about 10-50 bases in length occurs approximately 5oC below the melting temperature for a given duplex. Incubation at temperatures below the optimum may allow mismatched base sequences to hybridize and can therefore result in reduced specificity.

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another even though the one sequence differs merely by a single base. Finally, there can be intramolecular and intermolecular hybrids formed within a probe if there is sufficient self-complementarily. Such structures can be avoided through careful probe design. Computer programs are available to search for this type of interaction.

The present disclosure provides exemplary methods for constructing a recombinant host cell capable of expressing proteins comprising LP-48 polypeptides, said method comprising transforming or otherwise introducing into a host cell a recombinant DNA vector that comprises an isolated DNA sequence that encodes polypeptides comprising sequences as shown in SEQ ID NO:2. The preferred host cell is any eukaryotic cell which can accommodate high level expression of an exogenously introduced gene or protein, and that will incorporate said protein into its membrane structure. The skilled artisan understands that choosing the most appropriate cloning vector or expression vector depends upon a number of factors including the availability of restriction enzyme sites, the type of host cell into which the vector is to be transfected or transformed, the purpose of the transfection or transformation (e.g., stable transformation as an extrachromosomal element, or integration into the host chromosome), the presence or absence of readily assayable or selectable markers (e.g., antibiotic resistance and metabolic markers of one type and another), and the number of copies of the gene desired in the host cell.

When preparing an expression vector the skilled artisan understands that there are many variables to be considered, for example, whether to use a constitutive or inducible promoter. The practitioner also understands that the amount of nucleic acid or protein to be produced dictates, in part, the selection of the expression system. Regarding promoter sequences, inducible promoters are preferred because they enable high level, regulatable expression of an operably linked gene. The skilled artisan will recognize a number of suitable promoters that respond to a variety of inducers, for example, carbon source, metal ions, and heat. Other relevant considerations regarding an expression vector include whether to include sequences for directing the localization of a recombinant protein. For example, a sequence encoding a signal peptide preceding the coding region of a gene is useful for directing the extra-cellular export of a resulting polypeptide. Transformed host cells may be cultured under conditions well known to skilled artisans such that a polypeptide comprising sequence as shown in SEQ ID NO:2 is expressed, thereby producing a recombinant LP-48 protein in the recombinant host cell.

### Transgenic and Chimeric Non-Human Mammals

Nucleic acids which encode a LP-48 polypeptide of the present invention or any of its modified forms can also be used to generate either transgenic animals or "knock out" animals which, in turn, are useful in the development and screening of therapeutically useful reagents. Methods for generating transgenic animals, particularly animals such as mice or rats, have become conventional in the art and are described, for example, in U.S. Patent Nos. 4,736,866 and 4,870,009. Typically, particular cells would be targeted for an LP-48 transgene incorporation with tissue-specific enhancers. Transgenic animals that include a copy of a transgene introduced into the germ line of the animal at an embryonic stage can be used to examine the effect of increased expression of DNA encoding an LP-48 polypeptide. Such animals can be used as tester animals for reagents thought to confer protection from, for example, pathological conditions associated with its overexpression. Such an animal may be treated with the reagent and a reduced incidence of the pathological condition, compared to untreated animals bearing the transgene, would indicate a potential therapeutic intervention for the pathological condition.

Alternatively, non-human homologs of LP-48 can be used to construct a "knock out" animal which has a defective or altered gene encoding a particular LP-48 polypeptide as a result of homologous recombination between the endogenous gene encoding the LP-48 polypeptide and the altered genomic DNA introduced into an embryonic cell of the animal. For example, cDNA encoding an LP-48 polypeptide can be used to clone genomic DNA encoding that LP-48 polypeptide in accordance with established techniques. A portion of the genomic DNA encoding an LP-48 polypeptide can be deleted or replaced with another gene, such as a gene encoding a selectable marker that can be used to monitor integration. Typically, several kilobases of unaltered flanking DNA (both at the 5' and 3' ends) are included in the vector [see, e.g., Thomas and Capecchi, *Cell* 51(3): 503-12 (1987) for a description of homologous recombination vectors]. The vector is introduced into an embryonic stem cell line (e.g., by electroporation), and cells in which the introduced DNA has homologously recombined with the endogenous DNA are selected [see, e.g., Li et *al., Cell* 69(6): 915-26 (1992)]. The selected cells are then injected into a blastocyst of an animal (e.g., a mouse or rat) to form aggregation chimeras [see, e.g., Bradley, *Teratocarcinomas and Embryonic Stem Cells: A Practical Approach,* E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized, for instance, for their ability to defend against certain pathological conditions and for their development of pathological conditions due to absence of the native LP-48 polypeptide.

Transgenic non-human mammals are useful as an animal models in both basic research and drug development endeavors. Transgenic animals expressing at least one LP-48 polypeptide or nucleic acid can be used to test compounds or other treatment modalities which may prevent, suppress, or cure a pathology or disease associated with at least one of the above mentioned activities. Such transgenic animals can also serve as a model for the testing of diagnostic methods for those same diseases. Furthermore, tissues derived from such transgenic non-human mammals are useful as a source of cells for cell culture in efforts to develop *in vitro* bioassays to identify compounds that modulate LP-48 polypeptide activity or LP-48 polypeptide dependent signaling. One method of identifying compounds efficacious in the treatment of at least one previously described disease or pathology associated with a LP-48 polypeptide associated activity comprises:
a) generating a transgenic non-human animal which expresses a LP-48 polypeptide of the present invention and which is, as compared to a wild-type animal, pathologically distinct in some detectable or measurable manner from wild-type version of said non-human mammal;
b) exposing said transgenic animal to a compound, and;
c) determining the progression of the pathology in the treated transgenic animal, wherein an arrest, delay, or reversal in disease progression in transgenic animal treated with said compound as compared to the progression of the pathology in an untreated control animals is indicative that the compound is useful for the treatment of said pathology.

One method of identifying compounds capable of inhibiting LP-48 polypeptide activity *in vivo* and/or *in vitro* comprises:
a) administering an experimental compound to a LP-48 polypeptide expressing transgenic non-human animal, or tissues derived therefrom, exhibiting one or more physiological or pathological conditions attributable to the expression of a LP-48 transgene; and
b) observing or assaying said animal and/or animal tissues to detect changes in said physiological or pathological condition or conditions.

One method for identifying compounds capable of overcoming deficiencies in LP-48 polypeptide activity *in vivo* or *in vitro* comprises:
a) administering an experimental compound to a LP-48 polypeptide expressing transgenic non-human animal, or tissues derived therefrom, exhibiting one or more physiological or pathological conditions attributable to the disruption of the endogenous LP-48 polypeptide-encoding gene; and
b) observing or assaying said animal and/or animal tissues to detect changes in said physiological or pathological condition or conditions.

A compound's ability to modulate the activity of an LP-48 polypeptide in the body of the transgenic animal may be determined by various means. Observing the reversal of a pathological condition in the LP-48 polypeptide expressing transgenic animal after administering a compound is one such means. Another more preferred means is to assay for markers of LP-48 activity in the blood of a transgenic animal before and after administering an experimental compound to the animal. The level of skill of an artisan in the relevant arts readily provides the practitioner with numerous methods for assaying physiological changes related to therapeutic modulation of LP-48 activity.

"Gene therapy" includes both conventional gene therapies, where a lasting effect is achieved by a single treatment, and the administration of gene therapeutic agents, which involves the one time or repeated administration of a therapeutically effective DNA or mRNA. Anti-sense RNAs and DNAs can be used as therapeutic agents for blocking the expression of certain genes in vivo. It has already been shown that short anti-sense oligonucleotides can be imported into cells where they act as inhibitors, despite their low intracellular concentrations caused by their restricted uptake by the cell membrane [Zamecnik et al., *Proc. Natl. Acad Sci. USA* 83(12): 4143-6 (1986)]. The oligonucleotides can be modified to enhance their uptake, e.g., by substituting their negatively charged phosphodiester groups with uncharged groups.

There are a variety of techniques available for introducing nucleic acids into viable cells. The techniques vary depending upon whether the nucleic acid is transferred into cultured cell *in vitro* or *in vivo* in the cells of the intended host. Techniques suitable for the transfer of nucleic acid into mammalian cells *in vitro* include the use of liposomes, electroporation, microinjection, cell fusion, DEAE-dextran, the calcium phosphate precipitation method, etc. The currently preferred in vivo gene transfer techniques include transfection with viral (typically, retroviral) vectors and viral coat protein-liposome mediated transfection [Dzau et al., *Trends in Biotechnology* 11(5): 205-10 (1993)]. In some situations it is desirable to provide the nucleic acid source with an agent that targets the target cells, such as an antibody specific for a cell surface membrane protein or the target cell, a ligand for a receptor on the target cells, etc. Where liposomes are employed, proteins which bind to a cell surface membrane protein associated with endocytosis may by used for targeting and/or to facilitate uptake, e.g., capsid proteins or fragments thereof trophic for a particular cell type, antibodies for proteins which undergo internalization in cycling, proteins that target intracellular localization and enhance intracellular half-life. The technique of receptor-mediated endocytosis is described, for example by Wu et al., *J. Biol. Chem.* 262(10): 4429-32 (1987); and Wagner et al., *Proc. Natl. Acad. Sci. USA* 87(9): 3410-4 (1990). For a review of gene marking and gene therapy protocols, see Anderson, Science 256(5058): 808-13 (1992).

### Methods of Treatment Using LP-48 Polypeptides

As demonstrated in Examples 15 and 16, LP-48 polypeptides can significantly reduce apoptosis in human endothelial cells. In addition to reducing endothelial cell apoptosis, LP-48 polypeptides have been shown to bind to human endothelial cells (Example 17). Endothelial cell apoptosis has been found to occur in a number of diseases including, but not limited to, atherosclerosis, hypertension, congestive heart failure, primary pulmonary hypertension, sepsis, systemic inflammatory response syndrome, ARDS, multiple organ dysfunction, and allograft vasculopathy. The ability of LP-48 to reduce human endothelial cell apoptosis as well as bind to human endothelial cells indicates that LP-48 may prove useful in blocking the destruction of endothelial tissue that occurs through apoptosis. Accordingly, LP-48 may be used to treat atherosclerosis, hypertension, congestive heart failure, primary pulmonary hypertension, sepsis, systemic inflammatory response syndrome, ARDS, multiple organ dysfunction, allograft vasculopathy, and additional conditions and diseases in which endothelial cell apoptosis occurs.

Example 14 shows that LP-48 polypeptides are useful in inhibiting IFN-γ, IL-12, TNF-α,IL-6 and GM-CSF secretion, all of which are considered important in the pathogenesis of inflammatory and autoimmune diseases including, but not limited to, sepsis, gram negative bacteremia, allergic responses, allergic autoimmune diseases, type 1 diabetes, Th1-dependent insulitis, inflammation, multiple sclerosis, rheumatoid arthritis, inflammatory bowel disease, liver failure, ARDS, and conditions or symptoms related thereto. Furthermore, LP-48 polypeptide appears to bind to both mouse and human pancreatic cells (see Example 18). Because LP-48 polypeptides are useful in inhibiting IFN-γ and IL-12 production (see Example 14), LP-48 polypeptides can be used to block the destructive effects of IFN-γ, IL-12, IL-6, TNFα, and /or IL-1β in the pancreas and prevent pancreatic inlet cell apoptosis. Accordingly, LP-48 may be used to treat diabetes (Example 20).

Data presented in Examples 12 and 13 show that sepsis, gram negative bacteremia, acute inflammation, and conditions or symptoms related thereto may be treated or prevented by administration of effective amounts of LP-48 polypeptides. Administration of purified recombinant LP-48 protein inhibited the effects occurring during acute endotoxic shock and prevented death. As characterized generally, the invention also relates to methods of preventing or treating conditions caused or exacerbated by chronic inflammation including, but not limited to, allergic responses, allergic autoimmune diseases, type 1 diabetes, Th1-dependent insulitis, inflammation, multiple sclerosis, rheumatoid arthritis, inflammatory bowel disease, liver failure, ARDS, and conditions or symptoms related thereto by administering effective amounts of LP-48 polypeptides. The present invention further provides methods of preventing or treating additional inflammation mediated disorders by administering a therapeutically effective amount of LP-48 polypeptide. These inflammation mediated disorders include, but are not limited to acne, acute bronchitis, bronchiolitis, acute lung injury, allergic conjunctivitis, allergic rhinitis, allograft rejection, Alzheimer's disease, angina, apoptosis, adult respiratory distress syndrome (ARDS), asthma, atopic eczema, burns, bursitis, cerebritis, cholecystitis, chronic bronchitis including acute exacerbations of chronic bronchitis, cirrhosis, contact dermatitis, Crohn's disease, cystitis, dermatoses, eczema, erosive gastritis, esophagitis, gastritis, glomerulonephritis, graft-versus-host disease (GVHD), hepatitis, hypertension, interstitial pulmonary fibrosis, iritis, irritable bowel syndrome, mastocytosis, migraine, myasthenia gravis, myocardial ischemia, osteoarthritis, osteomyelitis, pancreatitis, pharyngitis, prostatitis, reperfusion injury, sarcoidosis, sepsis, septic shock, systemic inflammatory response syndrome, Sjogrens syndrome, systemic lupus erythematosus, tendonitis, tonsillitis, tubulointerstitial nephritis, ulcerative colitis, uveitis, chronic inflammation, and conditions or symptoms related thereto. As demonstrated in Example 21, LP-48 polypeptides may be used in the manufacture of a medicament for the treatment of liver disease. LP-48 prevented LPS and D-galactosamine-induced liver damage and protected 100% mice from liver failure and death.

Example 14 shows that LP-48 inhibits IFNγ, TNFα, IL-1β and IL-6 secretion. These proinflammatory cytokines play essential roles in pathogenesis of rheumatoid arthritis. Accordingly, LP-48 may be used to treat rheumatoid arthritis as demonstrated in Example 22.

Multiple sclerosis is an autoimmune disease. IFN-γ and IL-12 play critical roles in disease development and relapse. It has been established that blockage of IL-12 and IFN-γ using antibodies prevent EAE development in mice. LP-48 inhibits both IFN-γ and IL-12 (Example 14) and can, therefore, be used to treat multiple sclerosis as presented in Example 23.

In addition to treating multiple sclerosis, LP-48 polypeptides may be used in the manufacture of a medicament for treating or preventing autoimmune diseases generally. Accordingly, LP-48 polypeptides may be used to treat autoimmune disorders including, but not limited to, GVHD, rheumatoid arthritis, asthma, eczema, atopy, inflammatory bowel disease, vasculitis, psoriasis, insulin-dependent diabetes mellitus, pancreatis, psoriasis, cancer, multiple sclerosis, Hashimoto's thyroiditis, Graves disease, transplant rejection, systemic lupus erythematosus, autoimmune nephropathy, autoimmune hematopathy, idiopathic interstitial pneumonia, hypersensitivity pneumonitis, autoimmune dermatosis, autoimmune cardiopathy, autoimmune infertility, Behcet's disease, autoimmune gastritis, fibrosing lung disease, fulminant viral hepatitis B, fulminant viral hepatitis C, autoimmune hepatitis, chronic hepatitis, chronic cirrhosis, chronic glomeruonephritis, thrombotic thrombocytopenic purpura (TTP) and hemolytic uremic syndrome (HUS), aplastic anemia, myelodysplasia, multiple organ dysfunction syndrome (MODS), adult respiratory distress syndrome (ARDS), and conditions or symptoms related thereto.

As shown in Example 14, LP-48 polypeptides inhibit IFNγ, TNFα, IL-12 and IL-6 secretion. In accordance with the ability of LP-48 to inhibit these factors, an antagonist of LP-48 may be used in the treatment or prevention of a T cell mediated condition. Such T cell mediated conditions include, but are not limited to, immunodeficiency, HIV, HIV-induced lymphoma, HIV-induced AIDS, fulminant viral hepatitis B, fulminant viral hepatitis C, chronic hepatitis, chronic cirrhosis, cytoprotection during cancer treatment, recuperation from chemotherapy, recuperation from irradiation therapy, and conditions or symptoms related thereto.

IL-12 is a central mediator of the cell-mediated immune response in part through its activities on the development, proliferation, and activities of Th1 cells (Locksley, R.M., Proc. Natl. Acad. Sci. (USA), 90:5879 (1993); Trinchieri, G., Immunol. Today, 14:335 (1993); Scott, P., Science, 260:496 (1993); Hseih, C.S. et al., Science, 260:547 (1993)). These IL-12 activities are antagonized by factors which are associated with the development of uncommitted T helper cells into Th2 cells and mediation of the humoral immune response (e.g., IL-4 and IL-10; Locksley, R.M., Proc. Natl. Acad. Sci. (USA) 90:5879, (1993); Trinchieri, G., (1993); Scott, P., Science 260:496, (1993); and Hseih, C.S. et al., Science 260:547(1993)). As shown in Example 14, LP-48 is useful in inhibiting IL-12 secretion. Accordingly, LP-48 may be used in the treatment or prevention of a Th2 cell mediated condition. Conversely, administration of an antagonist of LP-48 provides for enhancing Th2 cell mediated immunity.

Apoptotic endothelial cells have been evidenced in both human and animal model atherosclerosis (Stefanec, Chest, 117:841 (2000)). Risk factors associated with atherosclerosis typically have been observed to cause endothelial apoptosis in vitro or increase the number of circulating endothelial cells in vivo. Oxidized low density lipoprotein, which induces several changes associated with the early stages of atherosclerosis, has been observed to induce endothelial cell apoptosis of human umbilical vein endothelial cells in vitro (Harada-Shiba et al., J. Biol. Chem., 273:9681 (1998)). Many therapies shown to have beneficial effects on atherosclerosis have been shown to have or are believed to have an antiapoptotic effect. Given the ability of LP-48 polypeptide to inhibit endothelial cell apoptosis, one embodiment of the invention provides for the use of LP-48 in the treatment of atherosclerosis.

A hallmark of hypertension is capillary rarefaction, in which there is a reduction in the number of capillaries per volume of tissue. According to the Goldblatt model of hypertension, endothelial apoptosis largely contributes to microvascular rarefaction (Stefanec, Chest, 117:841 (2000)). Hypertension in turn has been suggested to have a proapoptotic on some cell types based on in vitro studies. Furthermore, endothelial dysfunction and increased superoxide production caused by hypertension are indicative of endothelial apoptosis. Several means of treating hypertension may possess an antiapoptotic effect. Through the antiapoptotic effects of LP-48, one embodiment of the invention provides for the use of a LP-48 polypeptide in the manufacture of a medicament for treating hypertension and conditions associated with hypertension.

Studies with animal models directed to congestive heart failure indicate that endothelial apoptosis contributes to this disease. For example, in an animal model of aortic insufficiency in which hemodynamic abnormality was induced, an increase in circulating endothelial cells (which are indicative of apoptosis) was observed 28 days after the induction (Stefanec, Chest, 117:841 (2000)). In another example, a rat model of monocrotaline-induced right ventricular failure showed over a period of 30 days a progressive and significant increase in the number of apoptotic endothelial cells in skeletal muscle (Stefanec, Chest, 117:841 (2000)). This increase in apoptotic endothelial cells was accompanied by an increase in TNF and atrial natriuretic peptide, which along with angiotensin II may contribute to endothelial cell apoptosis in congestive heart failure. Thus one embodiment of the invention provides for the use of a LP-48 polypeptide in treating congestive heart failure by reducing endothelial apoptosis.

Several observations associated with primary pulmonary hypertension have suggested that endothelial cell apoptosis may contribute to the pathogenesis of this disease. In a rat model of pulmonary hypertension, apoptosis was observed in the endothelial cell layer of adult rat pulmonary artery prior to the onset of progressive pulmonary hypertension (Jones and Rabinovitch, Circ. Res., 79:1131 (1996)). In human primary pulmonary hypertension, increased serum levels of IL-1β, which may enhance endothelial cell apoptosis, have been detected (Stefanec, Chest, 117:841 (2000)). Along these lines, the development of primary pulmonary hypertension has been observed to be reduced by treatment with an IL-1 receptor antagonist in a rat model (Voelkel et al., Am. J. Respir. Cell Mol. Biol., 11:664 (1994)). Several treatments for primary pulmonary hypertension, including prostacyclin, calcium channel blockers, and angiotensin-converting enzyme have antiapoptotic effects on the endothelium (Stefanec, Chest, 117:841 (2000)). The ability of LP-48 to reduce or prevent apoptosis in endothelial cells therefore provides an embodiment of the invention in which LP-48 is used in the treatment of primary pulmonary hypertension.

Substantially purified, or pure preparations of LP-48 polypeptides and/or LP-48 epitope recognizing antibodies can be formulated into a pharmaceutically acceptable composition. For instance, a pharmaceutical formulation adapted for the treatment of an inflammation mediated disorder containing a LP-48 polypeptide or a fragment, analog, and/or homolog thereof is contemplated by the present invention. Additionally, a pharmaceutical formulation adapted for the treatment of immunodeficiencies containing a LP-48 antibody is contemplated. Such pharmaceutical formulations can be dosed in a fashion consistent with good medical practice, taking into account the clinical condition of the individual patient (especially the side effects of treatment with LP-48 polypeptides and/or LP-48 epitope recognizing antibodies alone), the site of delivery of the LP-48 polypeptides and/or LP-48 recognizing antibody compositions, the method of administration, the scheduling of administration, and other factors known to practitioners.

An effective amount of an LP-48 polypeptide or LP-48 epitope recognizing antibody will serve to prevent or treat at least one symptom of allergic responses, allergic autoimmune diseases, type 1 diabetes, Th1-dependent insulitis, immunodeficiencies, cancers, inflammation, infectious diseases, or will serve to modulate the biological activity of at least one natural ligand. An effective amount of LP-48 polypeptide and/or LP-48 recognizing antibody to prevent or treat at least one symptom may be determined by prevention or amelioration of adverse conditions or symptoms of the diseases being treated. The therapeutically effective amount of LP-48 polypeptide and/or LP-48 recognizing antibody for purposes herein is thus determined by such considerations. By delivery of graduating levels of LP-48 polypeptide and/or LP-48 recognizing antibody within a pharmaceutical composition, a clinician skilled in the art can determine the therapeutically effective dose of a LP-48 polypeptide and/or LP-48 epitope recognizing antibody for treatment or prevention of atherosclerosis, hypertension, congestive heart failure, primary pulmonary hypertension, sepsis, gram negative bacteremia, allergic responses, allergic autoimmune diseases, type 1 diabetes, Th1-dependent insulitis, immunodeficiencies, cancers, inflammation, infectious diseases. Such determinations are well known in the art and within the skill of the clinician in adjusting the therapeutically effective amount of LP-48 polypeptide and/or LP-48 recognizing antibody in a pharmaceutical composition accordingly. A therapeutically effective amount of LP-48 polypeptide and/or LP-48 recognizing antibody results in a measurable modulation of the biological activity associated with LP-48 polypeptide.

As a general proposition, the total therapeutically effective amount of LP-48 polypeptide and/or LP-48 recognizing antibody administered parentally per dose of a pharmaceutical composition will be in the range of about 1 µg/kg/day to 10 mg/kg/day of patient body weight. However, as noted above, this will be subject to therapeutic discretion. Preferably, this dose is at least 0.001 mg/kg/day, or at least 0.01 mg/kg/day, or at least 0.10 mg/kg/day, or at least 1.0 mg/kg/day.

As a further proposition, if given continuously, the LP-48 polypeptide and/or LP-48 recognizing antibody is typically administered at a dose rate of about 0.1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appear to vary depending on the desired effect.

Pharmaceutical compositions containing the LP-48 polypeptide and/or LP-48 recognizing may be administered using a variety of modes that include, but are not limited to, oral, rectal, intra-cranial, parenteral, intracisternal, intrathecal, intravaginal, intraperitoneal, intratracheal, intrabroncho-pulmonary, topical, transdermal (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include, but are not limited to, intravenous, intramuscular, intraperitoneal, intrasternal, subcutaneous and intraarticular injection and infusion. Implants comprising LP-48 polypeptide and/or LP-48 recognizing antibody also can be used.

LP-48 polypeptides and/or LP-48 recognizing antibodies are also suitably administered by sustained-release systems. Suitable examples of sustained-release compositions include semi-permeable polymer matrices, e.g., films, or microcapsules. Sustained-release matrices include polylactides (U.S. Pat. No. 3,773.919, EP 58,481), copolymers of L-glutamic acid and γ-ethyl-L-glutamate (Sidman et al., Biopolymers 22:547-556 (1983)), poly (2-hydroxyethyl methacrylate (Langer, R., et al., J. Biomed. Mater. Res. 15:167-277 (1981))), ethylene vinyl acetate (R. Langer et al., 1982) or poly-D-3-hydroxybutyric acid (EP 133,988).

Sustained-release LP-48 polypeptide and/or LP-48 recognizing antibody compositions also include liposomally entrapped LP-48 polypeptides. Liposomes containing LP-48 polypeptides are prepared by methods known per se (DE 3,218,121; Epstein et al., Proc. Natl. Acad. Sci. (USA) 82:3688-3692 (1985); Hwang et al., Proc. Natl. Acad. Sci. (USA) 77:4030-4034 (1980)); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal LP-48 polypeptide therapy.

For parenteral administration, the LP-48 polypeptide and/or LP-48 recognizing antibody may be formulated generally by mixing at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to polypeptides.

Generally, the formulations are prepared by contacting the LP-48 polypeptide uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes.

The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) polypeptides, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG.

The LP-48 polypeptide and/or LP-48 recognizing antibody is typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation of LP-48 polypeptide salts. Pharmaceutical compositions comprising LP-48 polypeptides and/or LP-48 recognizing antibodies to be used for therapeutic administration must be sterile. Sterility is readily accomplished by filtration through sterile filtration membranes (e.g., 0.2 micron membranes). Pharmaceutical compositions comprising LP-48 polypeptides and/or LP-48 epitope recognizing antibodies generally are placed into a container having a sterile access port, for example, an intravenous solution bag or vial having a stopper pierceable by a hypodermic injection needle.

Pharmaceutical compositions comprising LP-48 polypeptides and/or LP-48 recognizing antibodies ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous LP-48 polypeptide solution, and the resulting mixture is lyophilized. The infusion solution is prepared by reconstituting the lyophilized LP-48 polypeptide using bacteriostatic Water-for-Injection.

In addition, pharmaceutical compositions comprising LP-48 polypeptides and/or LP-48 epitope recognizing antibodies may be used in the the treatment or prevention of sepsis, gram negative bacteremia, allergic responses, allergic autoimmune diseases, type 1 diabetes, Th1-dependent insulitis, immunodeficiencies, cancers, inflammation, infectious diseases, and conditions or symptoms related thereto, wherein said composition further comprises other therapeutic compounds.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present application, including definitions, will control. In addition, the materials, methods and examples described herein are illustrative only and not intended to be limiting.

The following examples more fully describe the present invention.

### EXAMPLE 1: Cloning of LP-48 encoding Polynucleotides

LP-48 partial DNA sequence was identified from single EST sequence in the Incyte database of a human prostate cDNA library. The full-length cDNA clone was isolated from a human leukocyte library by PCR. LP-48 contains 179 amino acids and was initially characterized as having similarity to IL-17 since it is 20% identical to IL-17.

### Example 2: Northern Blot and RT-PCR analysis of LP-48 expression

Northern blot analysis is carried out to examine IL-17 gene expression in human tissues, using methods described in Current Protocols in Molecular Biology. A cDNA probe containing the entire nucleotide sequence of LP-48 polypeptide is labeled with 32P using the random prime(TM) DNA labeling system (Amersham Life Science), according to manufacturer's instructions. After labeling, the probe is purified using a CHROMA SPIN- 100™ column (Clontech Laboratories, Inc.), according to manufacturer's protocol number PT1200-1. The purified labeled probe is then used to examine various human tissues for IL-17 mRNA.

Multiple Tissue Northern (MTN) blots containing various human tissues (H) or human immune system tissues (IM) are obtained from Clontech and are examined with the labeled probe using ExpressHyb hybridization solution (Clontech) according to manufacturer's protocol number PT1190-1. Following hybridization and washing, the blots are mounted and exposed to film at -70°C overnight, and films developed according to standard procedures. The results show LP-48 expression was very low and was not detected in human brain, placenta, lung, liver, kidney, spleen, thymus, prostate, ovary, intestine, colon, stomach, thyroid, spinal cord, lymphnode, trachea, adrenal gland. Using techniques known by one skilled in the art, RT-PCR detected LP-48 expression in testis and leukocytes as well as fetal liver and kidney.

### Example 3: Expression of LP-48 Polypeptides in Mammalian Cells

A typical mammalian expression vector contains at least one promoter element, which mediates the initiation of transcription of mRNA, the polypeptide coding sequence, and signals required for the termination of transcription and polyadenylation of the transcript. Additionally, each mammalian expression vector may have enhancers, Kozak sequences and intervening sequences flanked by donor and acceptor sites for RNA splicing.

Highly efficient transcription can be achieved with the early and late promoters from SV40 and the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in providing LP-48 include, for example, vectors such as pIRES1neo, pRetro-Off, pRetro-On, PLXSN, or pLNCX (Clontech Labs, Palo Alto, CA), pcDNA3.1 (+/-), pcDNA/Zeo (+/-) or pcDNA3.1/Hygro (+/-) (Invitrogen), PSVL and PMSG (Pharmacia, Uppsala, Sweden), pRSVcat (ATCC 37152), pSV2dhfr (ATCC 37146) and pBC12MI (ATCC 67109). Mammalian host cells that could be used include human HeLa, 293, H9 and Jurkat cells, mouse NIH3T3 and C127 cells, Cos 1, Cos 7 and CV 1, quail QC1-3 cells, mouse L cells and Chinese hamster ovary (CHO) cells.

Alternatively, the desired LP-48 encoding DNA sequences can be expressed in stable cell lines that contain the DNA sequences for expressing each subunit once integrated into a chromosome(s). The co-transfection with a selectable marker such as dhfr, gpt, neomycin, or hygromycin allows the identification and isolation of the transfected cells as known in the art.

The transfected LP-48 polypeptide encoding DNA sequences can also be amplified to express large amounts of the encoded polypeptide. The DHFR (dihydrofolate reductase) marker is useful to develop cell lines that carry several hundred or even several thousand copies of the DNA sequences of interest. Another useful selection marker is the enzyme glutamine synthase (GS) (Murphy et al., Biochem. J. 227:277-279 (1991); Bebbington et al., Bio/Technology 10:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins and polypeptides.

The expression vectors pC1 and pC4 contain the strong promoter (LTR) of the Rous Sarcoma virus (Cullen et al., Molec. Cell. Biol. 5:438-447 (1985)) plus a fragment of the CMV-enhancer (Boshart et al., Cell 41:521-530 (1985)). Multiple cloning sites, e.g., with the restriction enzyme cleavage sites BamHI, XbaI and Asp718, facilitate the cloning of the sTNRF6 DNA sequences. The vectors contain, in addition to the 3' intron, the polyadenylation and termination signal of the rat preproinsulin gene.

293T cells can be transfected with a PvuI linearized expression plasmid using the calcium phosphate coprecipitation method. Neomycin clones can be selected in 400 µg/ml G418 and selected clones expanded. Producing clones can be selected using an enzyme-linked immunosorbet assay with anti-human IgG1 and Northern analysis with P32-labeled LP-48 specific DNA probe. Similarly, clones producing the LP-48-Fc product can be produced in COS or CHO cells.

### Example 4: Cloning and Expression of LP-48 Polypeptides in COS or CHO Cells

A plasmid for expressing LP-48 polypeptides is made by cloning a cDNA encoding LP-48 polypeptides into the expression vector pcDNAI/Amp or pcDNAIII (which can be obtained from Invitrogen, Inc.). The expression vector(s) pcDNAI/amp and pcDNA III contain: (1) an E. coli origin of replication effective for propagation in E. coli and other prokaryotic cells; (2) an ampicillin resistance gene for selection of plasmid-containing prokaryotic cells; (3) an SV40 origin of replication for propagation in eukaryotic cells; (4) a CMV promoter, a polylinker, an SV40 intron; (5) several codons encoding a hemagglutinin fragment (i.e., an "HA" tag to facilitate purification) or HIS tag (see, e.g., Ausubel, et al., ed., Current Protocols in Molecular Biology, John Wiley and Sons, NY (1987-1999)) followed by a termination codon and polyadenylation signal arranged so that a cDNA can be conveniently placed under expression control of the CMV promoter and operably linked to the SV40 intron and the polyadenylation signal by means of restriction sites in the polylinker. The HA tag corresponds to an epitope derived from the influenza hemagglutinin polypeptide as has been previously described (Wilson et al., Cell 37:767-778 (1984)). The fusion of the HA tag to the LP-48, allows easy detection and recovery of the recombinant polypeptide with an antibody that recognizes the HA epitope. pcDNAIII contains, in addition, the selectable neomycin marker.

A DNA fragment encoding an LP-48 polypeptide is separately cloned into the polylinker region of the vector so that recombinant polypeptide expression is directed by the CMV promoter. Insertion into the vector is optionally with or without the HA epitope. The plasmid construction strategy is as follows. An LP-48 polypeptide encoding DNA can be amplified using primers that contain convenient restriction sites. The PCR amplified LP-48 encoding DNA fragment and the pcDNAI/Amp vector are digested with suitable restriction enzyme(s) and then the LP-48 encoding DNA fragment is ligated to a digested vector. Each ligation mixture is transformed into E. coli strain SURE (available from Stratagene Cloning Systems, 11099 North Torrey Pines Road, La Jolla, CA 92037), and the transformed culture is plated on ampicillin media plates which then are incubated to allow growth of ampicillin resistant colonies. Plasmid DNA for each subunit is isolated from resistant colonies and examined by restriction analysis or other means for the presence of LP-48 encoding fragment.

For expression of LP-48 polypeptides, COS cells are co-transfected with an expression vector, as described above, using DEAE-DEXTRAN, as described, for instance, in Sambrook et al., Molecular Cloning: A Laboratory Manual, Cold Spring Laboratory Press, Cold Spring Harbor, New York (1989).
Cells are incubated under conditions suitable for expression of LP-48.

The LP-48-HA polypeptide is detected by radiolabeling and immunoprecipitation, using methods described in, for example, Harlow et al., Antibodies: A Laboratory Manual, 2nd Ed., Cold Spring Harbor Laboratory Press, Cold Spring Harbor, New York (1988). To this end, two days after transfection, the cells are labeled by incubation in media containing 35S-cysteine for 8 hours. The cells and the media are collected, and the cells are washed and lysed with detergent-containing RIPA buffer: 150 mM NaCl, 1% NP-40, 0.1% SDS, 0.5% DOC, 50 mM TRIS, pH 7.5, as described by Wilson et al., Cell 37:767-778 (1984). Proteins are precipitated from the cell lysate and from the culture media using an HA-specific monoclonal antibody. The precipitated protein is then analyzed by SDS-PAGE and autoradiography. An expression product of the expected size is seen in the cell lysate, which is not seen in negative controls.

The vector pC4 can be used for expression of LP-48. Plasmid pC4 is a derivative of the plasmid pSV2-dhfr (ATCC Accession No. 37146). The plasmid contains the mouse DHFR gene under control of the SV40 early promoter. Chinese hamster ovary (dhfr-) or other cells lacking dihydrofolate activity that are co-transfected with LP-48 plasmids can be selected by growing the cells in a selective medium (alpha minus MEM, Life Technologies) supplemented with the chemotherapeutic agent methotrexate (MTX). The amplification of the DHFR genes in cells resistant to methotrexate has been well documented (see, e.g., Alt et al., J. Biol. Chem. 253:1357-1370 (1978); Hamlin et al., Biophys. Acta 1097:107-143 (1990); and Page et al., Biotechnology 9:64-68 (1991)). Cells grown in increasing concentrations of MTX develop resistance to the drug by overproducing the target enzyme, DHFR, as a result of amplification of the DHFR gene. If DNA sequences are linked to the DHFR gene, it is usually co-amplified and over-expressed. It is known in the art that this approach can be used to develop cell lines carrying more than 1,000 copies of the amplified gene(s). Subsequently, when the methotrexate is withdrawn, cell lines are obtained which contain the amplified DNA sequences integrated into one or more chromosome(s) of the host cell.

Plasmid pC4 contains the strong promoter of the long terminal repeat (LTR) of the Rous Sarcoma virus (Cullen et al., Molec. Cell. Biol. 5:438-447 (1985) for expression of inserted gene sequences. PC4 additionally contains a fragment isolated from the enhancer of the immediate early gene of human cytomegalovirus (CMV) (Boshart et al., Cell 41:521-530 (1985)). Downstream of the promoter are BamHI, XbaI, and Asp718 restriction enzyme cleavage sites that allow integration of the DNA sequences. Behind these cloning sites the plasmid contains the 3' intron and polyadenylation site of the rat preproinsulin gene. Other high efficiency promoters can also be used for the expression, e.g., the human β-actin promoter, the SV40 early or late promoters or the long terminal repeats from other retroviruses, e.g., HIV and HTLVI. Clontech's Tet-Off and Tet-On gene expression systems and similar systems can be used to express LP-48, in a regulated way in mammalian cells (Gossen et al., Proc. Natl. Acad. Sci. (USA), 89:5547-5551 (1982)). For the polyadenylation of the mRNA other signals, e.g., from the human growth hormone or globin genes, can be used as well. Stable cell lines carrying the DNA sequences of LP-48 integrated into the chromosomes can also be selected upon co-transfection with a selectable marker such as gpt, G418 or hygromycin. It is advantageous to use more than one selectable marker in the beginning, e.g., G418 plus methotrexate.

The plasmid pC4 is digested with restriction enzymes and then dephosphorylated using calf intestinal phosphatase by procedures known in the art. The vector is then isolated from a 1% agarose gel. The DNA sequence encoding the complete LP-48 sequence is amplified using PCR oligonucleotide primers corresponding to the 5' and 3' sequences of the gene. Non-limiting examples include 5' and 3' primers having nucleotides corresponding or complementary to a portion of the coding LP-48 according to methods known in the art.

The amplified fragment(s) are digested with suitable endonucleases and then purified again on a 1% agarose gel. The isolated fragments for each subunit and the dephosphorylated vector are then separately ligated with T4 DNA ligase. *E. coli* HB101 or XL-1 Blue cells are then separately transformed and bacteria are identified that contain the fragment (or fragments if the vector is adapted for expressing both alpha and beta subunits) inserted into plasmid pC4 using, for instance, restriction enzyme analysis.

Chinese hamster ovary (CHO) cells lacking an active DHFR gene are used for transfection. 5 µg of the expression plasmid(s) pC4 is cotransfected with 0.5 µg of the plasmid pSV2-neo using lipofectin. The plasmid pSV2neo contains a dominant selectable marker, the neo gene from Tn5 encoding an enzyme that confers resistance to a group of antibiotics including G418. The cells are seeded in alpha minus MEM supplemented with 1 µg/ml G418. After 2 days, the cells are trypsinized and seeded in hybridoma cloning plates (Greiner, Germany) in alpha minus MEM supplemented with 10, 25, or 50 ng/ml of methotrexate plus 1 µg/ml G418. After about 10-14 days single clones are trypsinized and then seeded in 6-well petri dishes or 10 ml flasks using different concentrations of methotrexate (50 nM, 100 nM, 200 nM, 400 nM, 800 nM). Clones growing at the highest concentrations of methotrexate are then transferred to new 6-well plates containing even higher concentrations of methotrexate (1 mM, 2 mM, 5 mM, 10 mM, 20 mM). The same procedure is repeated until clones are obtained which grow at a concentration of 100 - 200 mM. Expression of the desired product is analyzed, for instance, by SDS-PAGE and Western blot or by reverse phase HPLC analysis.

### Example 5: Prokaryotic Expression and Purification of LP-48 Protein

An expression vector suitable for expressing LP-48 polypeptides or fragments thereof in a variety of prokaryotic host cells, such as E. coli, is easily made. The vector contains an origin of replication (Ori), an ampicillin resistance gene (Amp) useful for selecting cells which have incorporated the vector following a transformation procedure, and further comprises the T7 promoter and T7 terminator sequences in operable linkage to a LP-48 polypeptide encoding region. Plasmid pET11A (obtained from Novogen, Madison WI) is a suitable parent plasmid. pET11A is linearized by restriction with endonucleases NdeI and BamHI. Linearized pET11A is ligated to a DNA fragment bearing NdeI and BamHI sticky ends and comprising the coding region of the LP-48 gene as disclosed by SEQ ID NO:1 or a fragment thereof.

The LP-48 polypeptide encoding DNA used in this construction may be slightly modified at the 5' end (amino terminus of encoded protein) in order to simplify purification of the encoded protein product. For this purpose, an oligonucleotide encoding 8 histidine residues is inserted after the ATG start codon. Placement of the histidine residues at the amino terminus of the encoded protein serves to enable the IMAC one-step protein purification procedure.

An expression vector that carries an open reading frame (ORF) encoding a LP-48 polypeptide or fragment thereof and which ORF is operably-linked to an expression promoter is transformed into E. coli BL21 (DE3)(hsdS gal λcIts857 ind1Sam7nin51acUV5-T7gene 1) using standard methods. Transformants, selected for resistance to ampicillin, are chosen at random and tested for the presence of the vector by agarose gel electrophoresis using quick plasmid preparations. Colonies which contain the vector are grown in L broth and the protein product encoded by the vector-borne ORF is purified by immobilized metal ion affinity chromatography (IMAC), essentially as described in US Patent 4,569,794.

Briefly, the IMAC column is prepared as follows. A metal-free chelating resin (e.g., Sepharose 6B IDA, Pharmacia) is washed in distilled water to remove preservative substances and infused with a suitable metal ion [e.g., Ni(II), Co(II), or Cu(II)] by adding a 50mM metal chloride or metal sulfate aqueous solution until about 75% of the interstitial spaces of the resin are saturated with colored metal ion. The column is then ready to receive a crude cellular extract containing the recombinant protein product. After removing unbound proteins and other materials by washing the column with any suitable buffer, pH 7.5, the bound protein is eluted in any suitable buffer at pH 4.3, or preferably with an imidizole-containing buffer at pH 7.5.

### Example 6: Production of an Antibody to LP-48 Polypeptides or Fragments Thereof

A substantially pure LP-48 polypeptide or fragment thereof is isolated from transfected or transformed cells using any of the methods well known in the art, or by a method specifically disclosed herein. Concentration of protein in a final preparation is adjusted, for example, by filtration through an Amicon filter device such that the level is about 1 to 5 µg/ml. Monoclonal or polyclonal antibody can be prepared as follows.

Monoclonal antibodies can be prepared from murine hybridomas according to the method of Kohler and Milstein (Kohler and Milstein Nature 256:495 (1975)), or a modified method thereof. Briefly, a mouse is repetitively inoculated using a few micrograms of the peptide, polypeptide, or fusion polypeptide, over a period of a few weeks. The mouse is then sacrificed and the antibody producing cells of the spleen isolated. The spleen cells are fused by means of polyethylene glycol with mouse myeloma cells. Fused cells that produce antibody are identified by any suitable immunoassay, for example, ELISA, as described in E. Engvall, (Meth. Enzymol. 70:419 (1980)).

Polyclonal antiserum can be prepared by well-known methods (see, e.g., J. Vaitukaitis et. al., Clin. Endocrinol. Metab. 33:988 (1971)) that involve immunizing suitable animals with one or more of the LP-48 polypeptides fusion polypeptides, or fragments thereof, disclosed herein. Small doses (e.g., nanogram amounts) of antigen administered at multiple intradermal sites appears to be the most reliable method.

### Example 7: Construction of LP-48-Flag Expression Vector

To facilitate confirmation of LP-48 polypeptide expression (without the use of LP-48 epitope recognizing antibodies), a bicistronic expression vector (pIG1-LP-48F) is constructed by insertion of an "internal ribosome entry site"/enhanced green fluorescent protein (IRES/eGFP) PCR fragment into the mammalian expression vector pGTD (Gerlitz, B. et al. Biochemical Journal 295:131 (1993)). This new vector, designated pIG1, contains the following sequence landmarks: the E1a-responsive GBMT promoter (D. T. Berg et al., BioTechniques 14:972 (1993); D.T. Berg et al. Nucleic Acids Research 20:5485 (1992)); a unique BclI cDNA cloning site; the IRES sequence from encephalomyocarditis virus (EMCV); the eGFP (Clontech) coding sequence (Cormack, et al., Gene 173:33 (1996); the SV40 small "t" antigen splice site/poly-adenylation sequences; the SV40 early promoter and origin of replication; the murine dihydrofolate reductase (dhfr) coding sequence; and the pBR322 ampicillin resistance marker/origin of replication.

A pair of primers containing the DNA sequence cleaved by the restriction enzyme BclI at their 5' termini are synthesized so that when used to amplify the LP-48 polypeptide encoding DNA they incorporate the DNA sequence encoding the eight amino acid Flag epitope in-frame with the DNA sequences encoding LP-48 at the 3' terminus of the amplified product (Micele, R.M. et al., J. Immunol., Methods 167:279 (1994)). These primers are used to PCR amplify the LP-48 polypeptide encoding DNA. The resultant PCR product (LP-48F) is then digested with BclI (restriction sites incorporated into the primers) and ligated into the unique BclI site of pIG1 to generate the plasmid pIG1-LP-48F. The human LP-48 cDNA orientation and nucleotide sequence can be confirmed by restriction digest and double stranded sequencing of the insert.

In this fashion, an expression vector encoding a LP-48 fusion protein containing the FLAG polypeptide (SEQ ID NO:3) directly fused to the C-terminus of mature LP-48 polypeptide may be generated. SEQ ID NO:4, for instance, shows amino amino acids 19-197 of SEQ ID NO:2 fused to the eight amino acid FLAG tag polypeptide epitope (SEQ ID NO:3).

### Example 8: Construction of LP-48 non-Flag Expression Vector

In order to generate a non-Flagged expression vector (pIG1-LP-48), the 24-base DNA sequence encoding the eight amino acid FLAG epitope is deleted from the pIG1-LP-48F construct using the Quik Change mutagenesis kit (Stratagene). A 35-base primer, and its complement, with identity to the 19-base sequences flanking the FLAG sequence is synthesized and used to prime PCR using the plasmid as template. The PCR product is digested with DpnI restriction endonuclease to eliminate the parental DNA, and the digested product is transformed into Epicurean XLI-Blue E.coli cells. Ampicillin-resistant transformants are picked and the plasmid DNA is analyzed by restriction digestion. Precise deletion of the 24-base sequence is confirmed by DNA sequencing of pIG1-LP-48. In accordance with deleting the FLAG encoding sequence, pIG1-LP-48 encodes for the expression of the mature, 179 amino acid LP-48 polypeptide (amino acids 19-197 of SEQ ID NO:2).

### Example 9: Construction of LP-48 Immunoglobulin Fusion Proteins

### A. EXPRESSION OF LP-48-FC FUSION

LP-48 is prepared as a fusion protein coupled to an immunoglobulin constant region. The immunoglobulin constant region may contain genetic modifications including those that reduce or eliminate effector activity inherent in the immunoglobulin structure. (See, e.g., PCT Publication No. WO88/07089, published September 22, 1988). Briefly, PCR overlap extension is applied to join DNA encoding the LP-48 to DNA encoding the hinge, CH2 and CH3 regions of human IgG1. This is accomplished as described in the following subsections.

A DNA fragment corresponding to the DNA sequences encoding full length or portion of LP-48 was prepared by polymerase chain reaction (PCR) using primer pairs designed so as to amplify sequences encoding LP-48 having DNA sequence 5' to the ATG that was added to incorporate a NdeI site and a EcoR47III site. A cDNA encoding full length served as the template for amplifying LP-48. PCR amplification with these primers generated a DNA fragment that encoded a full length or portion of LP-48. The sequence of human IgG1 was obtained through Genbank (accession: HUMIGCC4; Takahashi et. al Cell 29: 671-679, 1982). This was compiled into exons and a region upstream of the natural hinge region chosen as the fusion site. The 5' primer was designed to include an overlap for the LP-48 amplicon. The 3' primer was complementary to the Fc region and incorporates the translation stop codon.

PCR reactions were prepared in 100 µl final volume composed of Pfu polymerase and buffer (Stratagene) containing primers (1 µM each), dNTPs (200 µM each), and 1ng of template DNA.

The resulting fragment was then cleaved with NheI and Eco47II which recognize the unique sites incorporated into the forward PCR primer of the first PCR reaction and the reverse PCR primer of the second PCR reaction. The digested fragment was then cloned into an expression vector, pJB02 that had also been treated with these restriction enzymes.

The cloning resulted in clones that contained the LP-48-Fc fusion. The sequence can be confirmed by DNA sequences.

### B. ISOLATION OF STABLE CLONES

First, 293 T cells were grown and a transient transfection utilizing lipofectamine (GIBCO-BRL) was performed. Characterization of the supernatant revealed a protein of the size one would expect for a dimer of the LP-48-Fc, thereby confirming the integrity of the construct. The expression of the protein was confirmed by a Western blot utilizing an antibody to human IgG1.

### Example 10: Large Scale Purification of LP-48 Polypeptides

Large-scale production of LP-48 polypeptides is effected by first growing stable LP-48 expressing clones in several 10-liter spinners. After reaching confluency, cells are further incubated for 2-3 more days to secrete maximum amount of LP-48 into the media. Media containing LP-48 polypeptide is adjusted to 0.1% CHAPS and concentrated in an Amicon ProFlux M12 tangential filtration system to 350 ml. The concentrated media is centrifuged at 19,000 rpm (43,000 x g) for 15 minutes and passed over a SP-5PW TSK-GEL column (21.5 mm x 15 cm; TosoHaas) at a flow rate of 8 ml/min. The column is washed with buffer A (20 mM MOPS, 0.1% CHAPS, pH 6.5) until the absorbency (280 nm) returns to baseline and the bound proteins are eluted with a linear gradient from 0.1 M-0.3 M NaCl (in buffer A) developed over 85 min. Fractions containing LP-48 polypeptide are pooled and passed over a (7.5 mm x 7.5 cm) Heparin-5PW TSK-GEL column equilibrated in buffer B (50 mM Tris, 0.1% CHAPS, 0.3 M NaCl, pH 7.0). The bound protein is eluted with a linear gradient from 0.3 M-1.0 M NaCl (in buffer B) developed over 60 min. Fractions containing LP-48 polypeptide are pooled and passed over a 1 cm x 15 cm Vydac C4 column equilibrated with 0.1% TFA/H20. The bound LP-48 polypeptide is eluted with a linear gradient from 0-100% CH3CN/0.1% TFA. Fractions containing LP-48 polypeptide are analyzed by SDS-PAGE and found to be greater than 95% pure and are dialyzed against 8 mM NaP04, 0.5 M NaCl, 10% glycerol, pH 7.4.

### Example 11: Production of Transgenic Mice Expressing LP-48

The LP-48 gene fragment was excised from a DHFR vector by Asc I and Sal I digestion and gel-purified. This fragment was then ligated into the MluI and XhoI sites of plasmid pLIV.7 (provided by John Taylor, The J. David Gladstone Institutes) generating plasmid pLIV7-LP48. Plasmid pLIV.7 is described by Fan, J. et al., where it was used to create plasmid pLivhHL1 (Fan,J. et al., Proc. Natl. Acad. Sci. 91:8724, (1994)). pLiv. 7 is identical to pLivhHL 1 with the HL (hepatic lipase) sequence removed and contains the apo E gene promoter/5' flanking region and a hepatic enhancer sequence referred to as the hepatic control region (HCR). For microinjection into embryos, a 6.5 kb DNA fragment encompassing the Apo E gene promoter-LP48-hepatic control region (HCR) fusion gene was excised from plasmid pLIV7-LP48 by digestion with SalI and SpeI and purified by gel electrophoresis and glass bead extraction.

Transgenic mice were generated using established techniques (Hogan, B. et al. (1986) *Manipulating the Mouse Embryo: A Laboratory Manual.* Cold Spring Harbor Laboratory, N.Y.) as modified by Fox and Solter (Fox and Solter, Mol. Cell. Biol. 8: 5470, (1988)). Briefly, the 6.5 kb DNA fragment encompassing the Apo E gene promoter-LP48-HCR fusion gene was microinjected into the male pronuclei of newly fertilized one-cell-stage embryos (zygotes) of the FVB/N strain. The embryos were cultured in vitro overnight to allow development to the two-cell-stage. Two-cell embryos were then transplanted into the oviducts of pseudopregnant CD-1 strain mice to allow development to term. To test for the presence of the transgene in the newborn mice, a small piece of toe was removed from each animal and digested with proteinase K to release the nucleic acids. A sample of the toe extract was subsequently subjected to PCR analysis using human LP48-specific primers to identify transgene-containing "founder" mice. Founder transgenic mice were bred to produce stable lines of transgenics having plasma levels of LP-48 polypeptides from 2ng/ml to 100 µg/ml depending on the line.

### Example 12: LP-48 Protects Against LPS-induced Septic Shock in Mice

This example demonstrates that LP-48 polypeptides can protect against LPS induced septic shock in mice. These data indicate that LP-48 polypeptides are useful in preventing and treating such conditions.

10-week old BALB/c mice (Harlan, Indianapolis) were given 175 µg/mouse of LPS by intravenous injection (the lateral tail vein) to induce sepsis and 1 hour later, human LP-48 polypeptide (50 µg), or (2) 50 µg BSA control was injected by intravenous, respectively. The survival rates of the mice were determined 24, 48 and 72 hours after LPS injection. LP-48 polypeptide demonstrated a relative survival rate of 100% in one experiment and 80% in another experiment. Injection of BSA showed no significant protection in this model. LP-48 polypeptide showed similar protection when LP-48 polypeptide was injected subcutaneously to mice 2 hours before LPS challenge.

### Example 13: LP-48 Transgenic Mice are Protected from LPS-induced Death.

Human LP-48 was expressed in FVB mice according to Example 11 and LP-48 protein was detectable by Elisa in LP-48 mice plasma. 175 µg of LPS was injected intravenously into LP-48 transgenic mice or age-matched FVB wild type mice. The survival rates of the mice were determined 24, 48 and 72 hours after LPS injection. LP-48 transgenic mice showed relative survival rate of 100% in two separate experiments.

### Example 14: LP-48 Polypeptides Inhibit LPS-induced Increases in IFN-γ, IL-12, TNF-α and IL-6 Secretion

During endotoxemia, cytokines are central players in the disease process (Dinarello, C.A., 1996, Curr. Top. Microbiol. Immunol.). The proinflammatory cytokines IL-12, IFN-γ and TNF-α are key cytokines of the generalized Shwartzman reaction (Ozmen, L.M. et al., 1994, J. Exp. Med. 180:907). TNF-α, IL-12 and IFN-γ are rapidly induced following LPS administration and these factors all contribute to endotoxin-induced lethality (Gutierrez-Ramos J. C. et al., 1997, Immunol. 135:3972). TNF-α is the primary mediator of mortality (Pfeffer, K., Cell 73: 457). IFN-γ appears to act by promoting increased responsiveness to TNF, in part by enhancing synthesis of both TNF and its receptors (Doherty, G.M., et al., 1992, J. Immunol. 149:1666). IL-12 also can play a critical role during endotoxin shock, primarily through its function as an inducer of IFN-γ (Eur. J. Immunol. 25:672,1995). 175 µg/mouse of LPS was injected to age matched FVB wild type mice or FVB LP-48 transgenic mice by intravenous. Such a dose of LPS caused acute inflammation in mice and 90% to 100% death rate based on our experiments. In response to LPS treatment, many pro-inflammatory cytokines were synthesized during the first several hours. Plasma was collected at 0, 1, 3, 6 and 9 hours post LPS injection. TNF-α, IFN-γ, IL-12, IL-6, IL-1β, GM-CSF, IL-10, and IL-18 levels were analyzed using traditional ELISA methods. The results demonstrated that LP-48 polypeptide reduced IFN-γ, IL-12, and GM-CSF significantly in mouse plasma. Moreover, TNF-α and IL-6 levels were moderately decreased in LP-48 mice compared to WT mice at 6 and/or 9 hours post LP-48 polypeptide treatment. In conclusion, LP-48 inhibits IL-12, IFN-γ, and GM-CSF secretion, and also decreases TNF-α and IL-6 levels in the LPS model. The evidence provided here demonstrates that administration of LP-48 polypeptides can inhibit inflammation and may be an effective therapy in treating human inflammatory diseases including but not limited to type I diabetes, multiple sclerosis, liver failure, septic shock, sepsis, systemic inflammatory response syndrome (SIRS), ischemia-reperfusion injury, endotoxin lethality, arthritis, lung injury, inflammatory bowel disease, Crohn's disease, ARDS, or any disease which is associated with over production of proinflammatory cytokines including IL-12, IFN-γ, GM-CSF, IL-6, and TNFα.

### Example 15: LP-48 Reduces Apoptosis in Human Endothelial Cells

Without being bound by any particular theory, the effects of LP-48 polypeptide in preventing LPS-induced septic shock (example 12) and death (example 13) may have occurred in part through inhibition of endothelial cell apoptosis. Studies of medical conditions consistent with sepsis and acute lung injury indicate that exposure to LPS may result in endothelial cell apoptosis in vivo (Stefanec, Chest 117:841 (2000)).

The effects of LP-48 polypeptide on the apoptosis of endothelial cells were examined in an assay of human umbilical vein epithelial cell (HUVEC) apoptosis. *In vitro,* many human endothelial cells are susceptible to apoptosis by TNFα only in the presence of RNA transcription inhibitors or protein synthesis inhibitors (such as cycloheximide). HUVEC survival following exposure to TNFα is dependent on synthesis of cytoprotective proteins. Protein synthesis inhibitors prevent synthesis of these proteins after exposure to IL-1, LPS, or TNFα (Zen et al., J. Biol. Chem. 274:28808 (1999); Polunovsky et al., Exp. Cell Res. 214:584 (1994)).

To prepare cells for an apoptosis assay, pooled human umbilical vein endothelial cells (HUVEC) (pool P180, lot #9F2003) (Clonetics, San Diego, CA) were grown in endothelial growth medium (EGM) with 2% FBS (Clonetics), and maintained at 37°C, 5% CO₂, 95% relative humidity. Following two to six cell passages, HUVEC were seeded at 1 X 10⁵ cells/well in 6-well tissue culture-treated dishes with complete EGM (3 ml/well).

After 24 hours of incubation, medium was changed in all wells (3 ml/well), and LP-48-FLAG fusion polypeptide (containing the FLAG epitope fused to the native c-terminus of LP-48, as described in Example 7) was added to appropriate wells at 100 or 200 ng/ml for a 16-hour incubation before apoptosis induction.

Apoptosis was induced by the addition of cycloheximide (CHX) and TNFα. CHX (ICN Biomedicals) prepared as a stock solution of 5 mg/ml in MeOH was administered to cells at 10 ug/ml, followed by incubation for 30 minutes. Following the incubation in CHX, the cells were administered recombinant human TNFα (R+D Systems, Minneapolis, MN) at 100 ng/ml for a 24 hour incubation. Apoptosis was induced in cells in the presence or absence of LP-48. Negative controls included untreated cells, CHX alone, and TNFα alone.

Following incubation with TNFα, the cellular medium (containing detached cells) was collected in 12 X 75 mm Falcon polypropylene round-bottom tubes. To harvest attached cells, 1 ml of pre-warmed trypsin/EDTA (0.25%/1mM; GIBCO BRL Life Technologies) was added to monolayers for a 3 minute incubation at room temperature. Trypsin was inactivated by adding 0.5 ml of pre-warmed Trypsin Neutralization Solution (TNS, Clonetics). Trypsinized cells were dispersed by pipetting up and down, then were pooled with the medium containing detached cells. Cells were centrifuged at 1000 rpm, 5 minutes, room temperature.

Apoptosis in the cells was analyzed by flow cytometry. Cell pellets were washed and vortexed in 400 ul cold Dulbecco's PBS (no calcium, no magnesium) (Life Technologies) containing 0.1% BSA. Cells were pelleted as above, and fixed by adding (and mixing well) 400 ul of Cytofix/Cytoperm (BD Pharmingen). Fixation was carried out on ice at 4°C for at least 45 minutes. After fixation, cells were pelleted and washed (with vortexing) in 300 ul of 1X PERMWASH (BD Pharmingen). Cells were again centrifuged and the supernatant was removed. Anti-active caspase 3 antibody solution (40 µl) (BD Pharmingen FITC-conjugated rabbit anti-active caspase 3, monoclonal, diluted 1:2 in 1 X PERMWASH) was applied to cell pellets. Cells were incubated with antibody at 4°C in the dark for 30 minutes to 2 hours. Cold PBS/BSA was then added (1 ml) for a final wash. After centrifugation, cells were resupended in 0.5 ml PBS/BSA and vortexed well prior to fluorescence activated cell sorting (FACS) analysis. FACS analysis was then performed on a COULTER^{™} EPICS XL-MCL Flow Cytometer (Coulter). Dead cells were gated out, and 10,000 events were recorded.

The results of the FACS analysis are shown in Table 2. The mean percent of anti-active caspase 3 staining (i.e., mean percent green fluorescence of duplicate samples minus background obtained from untreated, stained cells) is shown, along with standard error. Statistical comparisons were made (by using a t-test) between apoptotic cells with or without LP-48 pretreatment. HUVEC pre-treated with LP-48 showed a significant reduction in apoptosis compared to HUVEC either not treated with protein or HUVEC treated with a non-related, control protein (VEGF or LP111). These results demonstrate that LP-48 can reduce apoptosis in endothelial cells.

### Example 16: LP-48 Reduces Staurosporine Induced Apoptosis in Endothelial Cells

As described in Example 15, apoptosis of endothelial cells in vitro may be induced using cycloheximide and TNFα. Alternatively, endothelial cell apoptosis may be induced *in vitro* by other means including, but not limited to, use of staurosporine, oxidized low density lipoprotein, and/or ionizing radiation. For example, oxidized low density lipoprotein, which induces early changes associated with

**Table 2**

| LP-48 Reducess TNFalpha + CHX Induced Apoptosis in HUVEC | | |
|---|---|---|
| HUVEC Treatment Conditions | Percent Anti-active Caspase 3 staining (%) | Standard deviation |
| | | |
| CHX alone (10 ug/ml) | 0.285 | 0.156 |
| MeOH alone (6 ul) | 0.04 | 0.021 |
| TNFalpha 100 ng/ml | 0.4 | 0.233 |
| TNFalpha 100 ng/ml + CHX (10 ug/ml) | 10.365 | 0.283 |
| VEGF alone 100 ng/ml | -0.18 | 0.092 |
| VEGF (100 ng/ml) + TNFalpha 100 ng/ml + CHX (10 ug/ml) | 10.615 | 0.919 |
| LP-111 alone 1 ug/ml | 0.115 | 0.127 |
| LP-111 alone 50 ng/ml | 0.185 | 0.071 |
| LP-111 (1 ug/ml) + TNFalpha 100 ng/ml + CHX (10 ug/ml) | 9.765 | 0.000 |
| LP-111 (200 ng/ml) + TNFalpha 100 ng/ml + CHX (10 ug/ml) | 9.615 | 0.071 |
| LP-111 (50 ng/ml) + TNFalpha 100 ng/ml + CHX (10 ug/ml) | 9.185, | 0.255 |
| LP-48 alone (200 ng/ml) | 0.155 | 0.028 |
| LP-48 alone (100 ng/ml) | -0.085 | 0.042 |
| LP-48 (200 ng/ml) + TNFalpha 100 ng/ml + CHX (10 ug/ml) | 8.635 | 0.071 |
| LP-48 (100 ng/ml) + TNFalpha 100 ng/ml + CHX (10 ug/ml) | 6.37 | 0.417 |

atherosclerosis, has been used to induce apoptosis in HUVEC (Harada-Shiba et al., J. Biol. Chem. 273:9681 (1998)). Ionizing irradiation induces apoptosis *in vitro* in human microvascular and macrovascular cells in a manner that is enhanced by the presence of LPS (Eissner et al., Blood, 86:4184 (1995)). Ionizing radiation also may be used to induce apoptosis *in vivo.* In studies of intestinal radiation, irradiated mice were observed to undergo apoptosis in microvascular endothelial cells in response to ionizing radiation (Paris et al., Science 293:293 (2001)).

To further examine the ability of LP-48 to inhibit apoptosis in endothelial cells, LP-48 polypeptide was tested in an assay in which apoptosis was induced with staurosporine and analyzed using the APOPercentage^{™} apoptosis assay (Biocolor Ltd., Belfast, Northern Ireland). In this assay, HUVEC were grown as described above in Example 15, and were seeded at 3X10⁴ cells per well in a 96-well plate. LP-48-FLAG fusion polypeptide (containing the FLAG epitope fused to the native C-terminus of LP-48, as described in Example 7) was added to appropriate wells at 100 or 200 ng/ml for a 16-hour incubation before apoptosis induction. Apoptosis was induced by treating the cells with 1ug/ml staurosporine (Sigma) for 1 hour. Cells were prepared and stained according to the APOPecentage^{™} apoptosis assay per the manufacturer's instructions (Biocolor Ltd.,Belfast, Northern Ireland).

As shown in Table 3, HUVEC treated with LP-48 protein and staurosporine underwent significantly less apoptosis than did HUVEC treated with staurosporine in the absence of LP-48 protein. The results of this staurosporine apoptosis assay further demonstrate that LP-48 can inhibit endothelial cell apoptosis. These results, taken with the results of the TNFα and CHX apoptosis assay of Example 15, indicate

**Table 3**

| LP-48 Reduces Staurosporine Induced Apoptosis in HUVEC | | |
|---|---|---|
| HUVEC Treatment Conditions | Percent of Apoptosis (%) | Standard deviation |
| | | |
| Stain alone | 50.88 | 4.72 |
| Staurosporine (SS) alone | 98.99 | 0.49 |
| SS + LP-48 100 ng/ml | 29.40 | 3.32 |
| SS + LP-48 200 ng/ml | 30.89 | 3.32 |
| LP-48 alone | 22.30 | 3.09 |

**Table 4**

| Binding of LP-48 to HUVEC | | |
|---|---|---|
| HUVEC Treatment Conditions | ¹²⁵I-Protein A Bound (CPM) | Standard Deviation |
| | | |
| LP-48 + anti-LP-48 Abs + ¹²⁵I-Protein A | 5632.7 | 290.9 |
| LP-48 + Control Abs + ¹²⁵I-Protein A | 317.4 | 52.9 |
| anti-LP-48 Abs + ¹²⁵I-Protein A | 372.8 | 33.7 |
| ¹²⁵I-Protein A | 247.2 | 16.5 |

that LP-48 protein can generally inhibit endothelial cell apoptosis independent of the means of apoptosis induction.

The ability of LP-48 protein to inhibit endothelial cell apoptosis suggests that the ability of LP-48 protein to protect mice against LPS-induced septic shock may be attributed to the ability of LP-48 to reduce or prevent endothelial cell apoptosis. Administration of LP-48 polypeptide therefore may be an effective therapy in treating medical disorders that are characterized by endothelial cell apoptosis, including but not limited to, atherosclerosis, hypertension, congestive heart failure, primary pulmonary hypertension, sepsis, gram negative bacteremia, allergic responses, allergic autoimmune diseases, type 1 diabetes, Th1-dependent insulitis, inflammation, multiple sclerosis, rheumatoid arthritis, inflammatory bowel disease, liver failure, ARDS, allograft vasculopathy, and conditions or symptoms related thereto.

### Example 17: LP-48 Binds to the Cell Surface of Endothelial Cells

HUVEC were examined for the ability to bind LP-48 polypeptides in a binding assay utilizing LP-48 polypeptides and anti-LP-48 antibodies. LP-48 protein was expressed and purified according to Examples 7 and 10. HUVEC were grown as described in Example 15, and were seeded at 5 X 10⁴ cells per well in a 96 well plate. The cells were blocked with 4% horse serum in PBS, followed by incubation with 5 ug/ml LP-48 for two hours. The cells were next washed three times, and then incubated with anti-LP-48 antibodies or with control antibodies to an unrelated protein for 1 hour. The cells were then extensively washed, and antibody binding was detected by radioimmunoassay using ¹²⁵I-protein A.

As shown in Table 4, antibodies to LP-48 were specifically detected as binding to HUVEC that were incubated with LP-48 protein, whereas little to no binding was observed for antibodies to LP-48 that were applied to HUVEC not incubated with LP-48 protein. Also, control antibodies showed little to no binding to HUVEC in the presence or absence of LP-48 protein. These results demonstrate that LP-48 can specifically bind to the cell surface of HUVEC.

### Example 18: LP-48 Polypeptide Tissue Specific Binding

LP-48 polypeptides were used to screen for the presence of its receptor in human tissues. LP-48 protein was expressed and purified according to Examples 7 and 10, respectively. Human tissue sections were fixed with paraformaldehyde. For binding assays, tissues were blocked with 2% horse serum and incubated with 2, 10, or 20 µg/ml human LP-48 polypeptide for 2 hours. The slides were then washed 3X with PBS and next incubated with anti-LP-48 or anti-Flag antibodies. The slides were washed 3X with PBS and incubated with fluorescence conjugated secondary antibodies. Specific fluorescence intensity was represented after subtraction of value obtained for IgG negative control. LP-48 polypeptide specifically bound to pancreas, liver, spleen, thymus, lung, stomach, placenta, bladder, adipose, adrenal, thyroid, appendix, lymph node.

### Example 19: Identification of LP-48 Binding Proteins including Natural LP-48 Receptors

LP-48 polypeptides may be used to screen for molecules that bind to LP-48 or molecules to which LP-48 binds. The molecules that bind to LP-48 may be agonists or antagonists of LP-48. They include antibodies, oligonucleotides, protein (receptor), or small molecules.

For instance, flag-tagged-LP-48 can be incubated with cell lysates of cells suspected of expressing LP-48 receptors in buffer containing of 10 mM Tris, 150 mM NaCL, 2 mM EDTA, 0.5% NP-40 and proteinase inhibitors (one pill per 50 ml buffer, Boehringer Mannheim), 4°C for 4 hours. Anti-flag beads (Sigma) can be added and the mixture is incubated for an additional 4 hours. Complexes can be recovered by centrifugation, washed with 20 times bead volume of binding buffer and eluted in factions with Tris-Glycine buffer (PH2.5). A fraction of each faction can be separated by electrophoresis on a polyacrylamide gel. The gel can be silver stained according to manufacturer's instructions (silver staining kit from Novex; San Diego, CA). Pools having positive bands can be pooled together and concentrated. The pooled samples can be again separated on a denaturing polyacrlyamide gel and transferred to a PVDF membrane. Proteins that bind to LP-48 polypeptides specifically can then be identified by micro sequencing according to methods known to those skilled in the art.

Alternatively, LP48 receptor(s) can be identified using expression cloning (Kitamura, Intl. J. of Hematology, 67:351-359 (1998)).

### Example 20: Use of LP-48 Polypeptides to Treat Type I Diabetes

Female NOD/Bom mice can be purchased from Jackson Lab. (Maine) at 9 weeks of age and maintained in our animal facility under conventional conditions with standard diet. To accelerate diabetes development, mice are treated with Cyclophosphamide (250mg/kg i.p.) at 70 days of age. One group of mice receives 50 µg/mouse/day LP-48 by subcutaneous injection and another group of mice receives 50 µg/mouse/day BSA. Urinary glucose is analyzed daily, and hyperglycemia is detected by blood glucose determinations. Animals are generally regarded as diabetic when blood glucose levels are found to be above 16.7 mmol/liter as determined by hexokinase method. BSA treated mice are diagnosed with diabetes 10-11 days after Cyclophosphamide injection. Mice are killed on 1, 3, 6 and 9-day post Cyclophosphamide injection for pancreas and spleen analysis.

### Example 21: Use of LP-48 Polypeptides to Treat Liver Disease

BALB/c mice (Harlan, Indianapolis) per each experimental group (6 or 12 animals) were given intravenous injections (the lateral tail vein) of 6 mg of D (+)-Galactosamine (Sigma, 39F-0539) in 100 µl of PBS (GIBCOBRL) and 3 µg of Lipopolysaccharide β from E. coli 026:B6 (LPS) (Difco, 3920-25-2) in 100 µl of PBS. One hour later, the animals were given intravenous injections of either (1) LP-48 (50 µg) or (2) BSA (50 µg) control, respectively. The survival rates of the mice variously treated mice were determined 24 hours after LPS injection. LP-48 polypeptides protected 100% mice from LPS and D-galcotosamine induced death. In the same experiment, age-matched wild type mice had a 71% death rate. Treatment with BSA showed no significant protection in this model.

### Example 22: Use of LP-48 Polypeptides to Treat Rheumatoid Arthritis (RA)

According to the collagen-induced arthritis (CIA) model of RA, DBA/1 mice can be immunized with bovine type II collagen in adjuvant and treated daily after disease onset with either recombinant human LP-48 polypeptides or with saline. Mice are monitored for paw swelling and clinically scored. Histology analysis is also performed. Treatment of established CIA with LP-48 polypeptides may be effective in inhibiting paw swelling as well as disease progression as defined by clinical scoring cartilage.

### Example 23: Use of LP-48 Polypeptides to Treat Autoimmune Diseases Including Multiple Sclerosis

Rats are immunized by subcutaneous injection in the hind footpads with 0.1 ml of MBP epitope in PBS (1.5 mg/ml) and emulsified with an equal volume of CFA. One group of animals receives 2mg/kg/day LP-48 by subcutaneous injection, and another group receives control BSA. Rats are then monitored daily for clinical signs by an observer who is blind to the treatment protocol. EAE is scored as follows: 0, clinically normal; 1, flaccid tail; 2 hind limb paralysis; and 3, front and hind limb paralysis.

### Example 24: Use Of LP-48 Polypeptides to Treat Inflammatory Bowel Diseases.

Specific pathogen-free, 5-6 week-old male SJL/J mice are purchased from Jackson Lab. Trinitrobenzene Sulfonic Acid (TNBS) is obtained from Sigma-Aldrich. TNBS colitis is induced as described previously (Neurath, M.F., et al., 1995, J. Exp. Med. 182:1281-1290; Kitani,A. et al. 2000, J. Exp. Med. 192:41-52). Briefly, 1.5-2.0 mg of TNBS dissolved in 50% ethanol is administrated per rectum. Seven days later, mice lose weight continuously and show other clinical features of chronic colitis. For the study of prevention of induction of TNBS colitis, LP-48 is administrated by SC injection at 2 mg/kg/day 7 days later for a week. A control group consists of mice receiving ethanol without TNBS. The weight of each mouse is monitored every 24 hours, and mice are killed at multiple time points for assessment of histologic findings and cytokine production.

### Example 25: In vivo testing of LP-48 for treatment or prevention of ARDS

Runaway apoptosis and inflammation may lead to acute respiratory distress syndrome (ARDS) or, if multiple organs are involved, sepsis. ARDS is most often encountered with other serious illnesses. Thirty-eight percent (38%) of ARDS cases occur in sepsis patients. ARDS research efforts have focused on pro-inflammatory cytokines, specifically TNF-α, IL-1, IL-6, and IL-8, some of which are elevated during ARDS. Experimental treatments revolving around cytokine antagonism have included prostaglandin E1, anti-TNF, antioxidants, and antiproteases. Experimental therapies include administration of corticosteroids, ventilator therapy (PEEP), surfactant replacement therapy, and inhaled nitric oxide therapy. Unfortunately, little or no benefit has been observed clinically from any experimental treatments to date. At the present time there is no FDA approved pharmacological treatment for ARDS.

ARDS and sepsis are characterized by an overactivation of cytokine pathways where there is massive apoptosis and/or inflammation of cells in lungs and multiple organs, respectively. Rabbits exposed to hyperoxia (100% O₂) for 64 hours develop clinical symptoms that have been recognized as very similar to human ARDS. For example, one molecular endpoint of the hyperoxia model is increased alveolar permeability to solute, which can be quantitated. Therefore, to determine the usefulness of LP-48 polypeptides as a prophylactic (before challenge) and/or treatment (before, during and/or after challenge), rabbits can be challenged with hyperoxia to induce the ARDS symptomology, treated accordingly with LP-48 polypeptides, and then solute permeability across the alveolar epithelium can be measured. LP-48 polypeptides of varying concentrations are generally given at different times. Thus, according to one embodiment of the present invention, LP-48 polypeptides may be useful in improving lung function in sepsis and/or ARDS patients and measures of lung function may include, but are not limited to, fluid transport across the alveoli.

### Example 26: Assays for T Cell Proliferation and Activity

Functional activity of LP-48 polypeptides and/or effects of LP-48 recognizing antibodies can be measured in a appropriately modified T cell proliferation and cytokine secretion assay. Essentially purified T cells from the spleen or lymph nodes (4x10⁵) in 200 µl RPMI and 10% FBS media are seeded in triplicate in 96 well plates that are coated with different concentrations of anti-CD3/anti-CD28, ConA, PHA, or PMA ionomycin in the presence or absence of LP-48. After 24, 48, or 72 hours, supernatants are collected for cytokine analysis and cells are pulsed for 12 hours with 1µCi of [3H] thymidine. Thymidine incorporation is quantified using a scintillation counter.

### Example 27: Assays for Macrophage Proliferation and Cytokine Secretion

Functional activity of LP-48 polypeptides and/or effects of LP-48 recognizing antibodies can be measured in an appropriately modified macrophage proliferation and cytokine secretion assay. Essentially purified macrophages from the peritoneum, spleen, or liver (1 x 10⁵) in 200 µl RPMI and 10% FBS media are seeded in triplicate in 96 well plates with different concentrations of LPS in the presence or absence of LP-48. After 24, 48, or 72 hours, supernatants are collected for cytokine analysis and cells are pulsed for 12 hours with 1µCi of [3H] thymidine. Thymidine incorporation is quantified using a scintillation counter.

### Example 28: Effect of LP-48 Polypeptides on T cell Priming and Cytokine Production in Wildtype and LP-48 Transgenic Mice

Functional activity of LP-48 polypeptides and/or effects of LP-48 recognizing antibodies can be measured in an appropriately modified T cell priming and cytokine production assay. Essentially, six-week-old WT and LP-48 transgenic mice (four mice in each group) are immunized with 100 µg KLH or HEL in complete Freunds adjuvant (CFA) in the hind footpads. CD4+ T cells are purified from the draining lymph nodes and cultured in the presence of antigen presenting cells (APC), different concentrations of KLH or HEL, and in the presence or absence of LP-48. APCs are isolated from the spleens of 6-wk-old WT and LP-48 transgenic mice and irradiated (3,000 rads). T cell recall responses are thereby tested. Cytokine secretion by CD4+ T cells from KLH immunized mice are assayed by ELISA.

To determine if the mechanism of response seen with LP-48 treatment occurs at the level of the APC or intrinsic hyperproliferative response of CD4+ T cells, KLH-primed CD4+ T cells purified from LP-48 transgenic and wild type mice are examined for their recall response in the presence of wild type or LP-48 transgenic APC.

The culture supernatants of T cell recall responses are assayed by ELISA for cytokine production. The production levels of Th1 and Th2 cytokines are observed in order to determine if LP-48 is critically involved in both the Th1 and Th2 differentiation during recall responses.

## Claims

1. Use of a polypeptide comprising amino acids 19-197 of the LP-48 as shown in SEQ ID NO: 2 in the manufacture of a medicament for treating or preventing an inflammation mediated disorder in a mammal, wherein said disorder is selected from sepsis, septic shock, systemic inflammatory response syndrome and acute respiratory distress syndrome (ARDS).

2. Use of a polypeptide comprising amino acids 19-197 of the LP-48 as shown in SEQ ID NO: 2 in the manufacture of a medicament for treating or preventing a disorder associated with endothelial cell apoptosis in a mammal, wherein said disorder is selected from atherosclerosis, hypertension, congestive heart failure, primary pulmonary hypertension, acute respiratory distress syndrome (ARDS) and allograft vasculopathy.

3. Use according to either claim 1 or claim 2 wherein said polypeptide comprises an LP-48 amino acid sequence as shown in SEQ ID NO:2.

4. Use according to any one of claims 1 to 3 wherein said polypeptide is a fusion protein comprising amino acids 19-197 of the LP-48 as shown in SEQ ID NO: 2 and an immunoglobulin constant domain sequence.

5. Use according to claim 4 wherein the LP-48 polypeptide comprises an amino acid sequence as shown in SEQ ID NO:2.

## Patentansprüche

1. Verwendung eines Polypeptids, das die Aminosäuren 19 bis 197 des LP-48 umfasst, wie sie in SEQ ID Nr. 2 gezeigt sind, zur Herstellung eines Arzneimittels zur Behandlung oder Prävention einer durch Entzündung vermittelten Störung bei einem Säuger, worin die Störung ausgewählt ist aus Sepsis, septischem Schock, systemisch entzündlichem Response-Syndrom und akutem Atemstresssyndrom (ARDS).

2. Verwendung eines Polypeptids, das die Aminosäuren 19 bis 197 des LP-48 umfasst, wie sie in SEQ ID Nr. 2 gezeigt sind, zur Herstellung eines Arzneimittels zur Behandlung oder Prävention einer Störung, die mit der Endothelzellapoptose bei einem Säuger assoziiert ist, worin die Störung ausgewählt ist aus Artherosklerose, Hypertension, kongestivem Herzversagen, primärer, pulmonaler Hypertension, akutem Atemstresssyndrom (ARDS) und einer Allotransplantatvaskulopathie.

3. Verwendung nach Anspruch 1 oder Anspruch 2, worin das Polypeptid eine LP-48 Aminosäuresequenz umfasst, wie sie in SEQ ID Nr. 2 gezeigt ist.

4. Verwendung nach einem der Ansprüche 1 bis 3, worin das Polypeptid ein Fusionsprotein ist, das die Aminosäuren 19 bis 197 des LP-48, wie sie in SEQ ID Nr. 2 gezeigt sind, und eine Sequenz der konstanten Domäne des Immunglobulins umfasst.

5. Verwendung nach Anspruch 4, worin das LP-48 Polypeptid die Aminosäuresequenz umfasst, wie sie in SEQ ID Nr. 2 gezeigt ist.

## Revendications

1. Utilisation d'un polypeptide comprenant les acides aminés 19 à 197 du LP-48 tels que représentés dans la SEQ ID NO: 2, dans la fabrication d'un médicament pour traiter ou prévenir un trouble médié par une inflammation chez un mammifère, dans laquelle ledit trouble est choisi parmi une septicémie, un choc septique, un syndrome de réponse inflammatoire systémique et un syndrome de détresse respiratoire aiguë (SDRA).

2. Utilisation d'un polypeptide comprenant les acides aminés 19 à 197 du LP-48 tels que représentés dans la SEQ ID NO: 2, dans la fabrication d'un médicament pour traiter ou prévenir un trouble associé à une apoptose des cellules endothéliales chez un mammifère, dans laquelle ledit trouble est choisi parmi une athérosclérose, une hypertension, une insuffisance cardiaque congestive, une hypertension pulmonaire primitive, un syndrome de détresse respiratoire aiguë (SDRA) et une vasculopathie d'allogreffe.

3. Utilisation selon la revendication 1 ou 2, dans laquelle ledit polypeptide comprend une séquence d'acides aminés de LP-48 telle que représentée dans la SEQ ID NO : 2.

4. Utilisation selon l'une quelconque des revendications 1 à 3, dans laquelle ledit polypeptide est une protéine de fusion comprenant les acides aminés 19 à 197 du LP-48 tels que représentés dans la SEQ ID NO : 2 et une séquence du domaine constant des immunoglobulines.

5. Utilisation selon la revendication 4, dans laquelle le polypeptide LP-48 comprend une séquence d'acides aminés telle que représentée dans la SEQ ID NO : 2.


SEQUENCE LISTING
<110> ELI LILLY AND COMPANY
<120> Methods of using A Human IL-17 Related polypeptide To Treat Disease
<130> X-14089
<160> 4
<170> PatentIn version 3.1
<210> 1
<211> 591
<212> DNA
<213> Homo sapiens
<220>
<221> CDS
<222> (1)..(591)
<223>
<400> 1
   atg acg ctc ctc ccc ggc ctc ctg ttt ctg acc tgg ctg cac aca tgc      48
   Met Thr Leu Leu Pro Gly Leu Leu Phe Leu Thr Trp Leu His Thr Cys
   1               5                   10                  15
   ctg gcc cac cat gac ccc tcc ctc agg ggg cac ccc cac agt cac ggt      96
   Leu Ala His His Asp Pro Ser Leu Arg Gly His Pro His Ser His Gly
               20                  25                  30
   acc cca cac tgc tac tcg gct gag gaa ctg ccc ctc ggc cag gcc ccc     144
   Thr Pro His Cys Tyr Ser Ala Glu Glu Leu Pro Leu Gly Gln Ala Pro
           35                  40                  45
   cca cac ctg ctg gct cga ggt gcc aag tgg ggg cag gct ttg cct gta     192
   Pro His Leu Leu Ala Arg Gly Ala Lys Trp Gly Gln Ala Leu Pro Val
       50                  55                  60
   gcc ctg gtg tcc agc ctg gag gca gca agc cac agg ggg agg cac gag     240
   Ala Leu Val Ser Ser Leu Glu Ala Ala Ser His Arg Gly Arg His Glu
   65                  70                  75                  80
   agg ccc tca gct acg acc cag tgc ccg gtg ctg cgg ccg gag gag gtg     288
   Arg Pro Ser Ala Thr Thr Gln Cys Pro Val Leu Arg Pro Glu Glu Val
                   85                  90                  95
   ttg gag gca gac acc cac cag cgc tcc atc tca ccc tgg aga tac cgt     336
   Leu Glu Ala Asp Thr His Gln Arg Ser Ile Ser Pro Trp Arg Tyr Arg
               100                 105                 110
   gtg gac acg gat gag gac cgc tat cca cag aag ctg gcc ttc gcc gag     384
   Val Asp Thr Asp Glu Asp Arg Tyr Pro Gln Lys Leu Ala Phe Ala Glu
           115                 120                 125
   tgc ctg tgc aga ggc tgt atc gat gca cgg acg ggc cgc gag aca gct     432
   Cys Leu Cys Arg Gly Cys Ile Asp Ala Arg Thr Gly Arg Glu Thr Ala
       130                 135                 140
   gcg ctc aac tcc gtg cgg ctg ctc cag agc ctg ctg gtg ctg cgc cgc     480
   Ala Leu Asn Ser Val Arg Leu Leu Gln Ser Leu Leu Val Leu Arg Arg
   145                 150                 155                 160
   cgg ccc tgc tcc cgc gac ggc tcg ggg ctc ccc aca cct ggg gcc ttt     528
   Arg Pro Cys Ser Arg Asp Gly Ser Gly Leu Pro Thr Pro Gly Ala Phe
                   165                 170                 175
   gcc ttc cac acc gag ttc atc cac gtc ccc gtc ggc tgc acc tgc gtg     576
   Ala Phe His Thr Glu Phe Ile His Val Pro Val Gly Cys Thr Cys Val
               180                 185                 190
   ctg ccc cgt tca gtg                                                 591
   Leu Pro Arg Ser Val
           195
<210> 2
<211> 197
<212> PRT
<213> Homo sapiens
<400> 2
   Met Thr Leu Leu Pro Gly Leu Leu Phe Leu Thr Trp Leu His Thr Cys
   1               5                   10                  15
   Leu Ala His His Asp Pro Ser Leu Arg Gly His Pro His Ser His Gly
               20                  25                  30
   Thr Pro His Cys Tyr Ser Ala Glu Glu Leu Pro Leu Gly Gln Ala Pro
           35                  40                  45
   Pro His Leu Leu Ala Arg Gly Ala Lys Trp Gly Gln Ala Leu Pro Val
       50                  55                  60
   Ala Leu Val Ser Ser Leu Glu Ala Ala Ser His Arg Gly Arg His Glu
   65                  70                  75                  80
   Arg Pro Ser Ala Thr Thr Gln Cys Pro Val Leu Arg Pro Glu Glu Val
                   85                  90                  95
   Leu Glu Ala Asp Thr His Gln Arg Ser Ile Ser Pro Trp Arg Tyr Arg
               100                 105                 110
   Val Asp Thr Asp Glu Asp Arg Tyr Pro Gln Lys Leu Ala Phe Ala Glu
           115                 120                 125
   Cys Leu Cys Arg Gly Cys Ile Asp Ala Arg Thr Gly Arg Glu Thr Ala
       130                 135                 140
   Ala Leu Asn Ser Val Arg Leu Leu Gln Ser Leu Leu Val Leu Arg Arg
   145                 150                 155                 160
   Arg Pro Cys Ser Arg Asp Gly Ser Gly Leu Pro Thr Pro Gly Ala Phe
                   165                 170                 175
   Ala Phe His Thr Glu Phe Ile His Val Pro Val Gly Cys Thr Cys Val
               180                 185                 190
   Leu Pro Arg Ser Val
           195
<210> 3
<211> 8
<212> PRT
<213> Artificial/unknown
<220>
<221> PEPTIDE
<222> (1)..(8)
<223> Flag epitope
<400> 3
   Asp Tyr Lys Asp Asp Asp Asp Lys
   1 5
<210> 4
<211> 187
<212> PRT
<213> Homo sapiens
<400> 4
   His His Asp Pro Ser Leu Arg Gly His Pro His Ser His Gly Thr Pro
   1 5 10 15
   His Cys Tyr Ser Ala Glu Glu Leu Pro Leu Gly Gln Ala Pro Pro His
   20 25 30
   Leu Leu Ala Arg Gly Ala Lys Trp Gly Gln Ala Leu Pro Val Ala Leu
   35 40 45
   Val Ser Ser Leu Glu Ala Ala Ser His Arg Gly Arg His Glu Arg Pro
   50 55 60
   Ser Ala Thr Thr Gln Cys Pro Val Leu Arg Pro Glu Glu Val Leu Glu
   65 70 75 80
   Ala Asp Thr His Gln Arg Ser Ile Ser Pro Trp Arg Tyr Arg Val Asp
   85 90 95
   Thr Asp Glu Asp Arg Tyr Pro Gln Lys Leu Ala Phe Ala Glu Cys Leu
   100 105 110
   Cys Arg Gly Cys Ile Asp Ala Arg Thr Gly Arg Glu Thr Ala Ala Leu
   115 120 125
   Asn Ser Val Arg Leu Leu Gln Ser Leu Leu Val Leu Arg Arg Arg Pro
   130 135 140
   Cys Ser Arg Asp Gly Ser Gly Leu Pro Thr Pro Gly Ala Phe Ala Phe
   145 150 155 160
   His Thr Glu Phe Ile His Val Pro Val Gly Cys Thr Cys Val Leu Pro
   165 170 175
   Arg Ser Val Asp Tyr Lys Asp Asp Asp Asp Lys
   180 185